# FASCICULE DE BREVET EUROPEEN

(11) **EP 1 527 068 B1**
(45) Date de publication et mention de la délivrance du brevet: **09.01.2008**
(21) Numéro de dépôt: 03755628.9
(22) Date de dépôt: 24.07.2003
(51) Int. Cl.: C07D 409/12, A61K 31/417, A61P 25/00, C07D 409/14

(54) **DERIVES D'ARYLIMIDAZOLES ET LEUR UTILISATION EN TANT QU'INHIBITEURS DE NO SYNTHASES ET EN TANT QUE MODULATEURS DES CANAUX SODIQUES**
ARYLIMIDAZOLDERIVATE UND DEREN VERWENDUNG ALS NO SYNTHASE INHIBITOREN UND ALS MODULATOREN DER NA-KANÄLE
NOVEL ARYLIMIDAZOLE DERIVATIVES, THEIR USE AS NO-SYNTASE INHIBITORS AND AS MODULATORS OF SODIUM CHANNELS

(30) Priorité: 25.07.2002 FR 0209416
(43) Date de publication de la demande: 04.05.2005
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Dennis, F-91190 Gif SUR YVETTE (FR); LIBERATORE, Anne-Marie, F-78610 Auffargis (FR); CHABRIER DE LASSAUNIERE, Pierre-Etienne, F-75016 Paris (FR)
(74) Mandataire: Bourgouin, André
(86) Numéro de dépôt international: PCT/FR2003/002336
(87) Numéro de publication internationale: WO 2004/011402

(56) Documents cités:
- WO-A-01/26656
- WO-A-98/58934

## Description

La présente demande concerne de nouveaux dérivés d'arylimidazoles, lesquels peuvent être utilisés en tant qu'inhibiteurs de NO synthases (NOS) et en tant que modulateurs des canaux sodiques.

Compte tenu du rôle potentiel du NO et des canaux sodiques en physiopathologie, les nouveaux dérivés décrits répondant à la formule générale **(I)** décrite plus loin peuvent produire des effets bénéfiques ou favorables :
❖ dans le traitement ou la prévention de la douleur, et notamment :
   ◆ des douleurs neuropathiques et en particulier :
      - des douleurs neuropathiques d'origine métabolique (comme par exemple les neuropathies diabétiques),
      - des douleurs neuropathiques d'origine infectieuse comme celles liées à des maladies virales ou rétrovirales (telles que par exemple les douleurs liées à l'herpès comme la douleur post-herpétique, les douleurs liées au Syndrome Immuno-Déficitaire Acquis (SIDA) ou les douleurs liées au zona),
      - des douleurs neuropathiques d'origine traumatique (comme par exemple celles liées à un membre fantôme)
   ◆ des névralgies glosso-pharyngiennes, des radiculopathies et neuropathies secondaires à des infiltrations métastatiques, de l'adiposis dolorosa et des douleurs liées aux brûlures,
   ◆ de la migraine,
   ◆ des douleurs post-opératoires,
   ◆ des douleurs centrales consécutives aux accidents cérébraux vasculaires, aux lésions thalamiques ou à la sclérose en plaques,
   ◆ des douleurs chroniques, et
   ◆ des douleurs liées à un cancer ;
❖ dans le traitement de la sclérose en plaques ;
❖ dans le traitement des troubles du système nerveux central ou périphérique et en particulier :
   ◆ de l'épilepsie,
   ◆ des maladies neurodégénératives parmi lesquelles l'on peut notamment citer les démences séniles y compris la maladie d'Alzheimer, la chorée de Huntington, la maladie de Parkinson, la sclérose latérale amyotrophique, l'ataxie de Freiderich et les maladies à prions (en particulier la maladie de Creutzfeld Jacob),
   ◆ de l'ischémie cérébrale et des traumatismes cérébraux ou de la moelle épinière,
   ◆ de la dépression et des troubles bipolaires,
   ◆ des encéphalopathies, y compris les encéphalopathies d'origine virale ou toxique,
   ◆ de l'addiction aux opiacées, à l'alcool et aux substances induisant une accoutumance,
   ◆ des troubles de l'érection et de la reproduction,
   ◆ des désordres cognitifs,
   ◆ de l'anxiété, de la schizophrénie, des troubles du sommeil et des troubles alimentaires (anorexie, boulimie, etc.) ;
❖ dans le traitement des troubles cardiovasculaires comme les infarctus cardiaques ou des troubles du rythme cardiaque, plus particulièrement de l'arythmie ;
❖ dans le traitement des troubles du muscle squelettique et des jonctions neuromusculaires comme les myopathies ;
❖ dans le traitement des maladies inflammatoires comme par exemple le psoriasis, l'arthrose et l'arthrite rhumatoïde, les inflammations du système gastro-intestinal (colite, maladie de Crohn) ou du système pulmonaire et des voies aériennes (asthme, sinusites, rhinites) ainsi que les hypersensibilités de contact ou retardées, et en particulier l'arthrose et l'arthrite rhumatoïde ;
❖ dans le traitement des pertes d'audition d'origine traumatique, acoustique ou toxique et des acouphènes.
❖ dans le traitement des complications liées aux maladies auto-immunes et virales comme par exemple le lupus, le sida, les infections parasitaires et virales, les complications du diabète incluant les rétinopathies, les néphropathies et les polyneuropathies ;
❖ dans le traitement des maladies neurologiques associées à des intoxications (empoisonnement au Cadmium, inhalation de *n*-hexane, pesticide, herbicide), à des traitements (radiothérapie) ou à des désordres d'origine génétique (maladie de Wilson) ;
❖ et plus généralement dans le traitement de toutes les pathologies caractérisées par une production excessive de monoxyde d'azote et/ou ou un dysfonctionnement des canaux sodiques.

La demanderesse avait décrit dans la demande WO 01/26656 des dérivés d'imidazoles pouvant moduler les canaux sodiques, à savoir les composés de formule générale **(A1)** 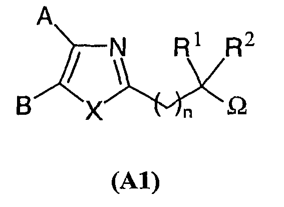 dans laquelle :
A représente (notamment) un radical phényle ou biphényle éventuellement substitué ;
B représente (notamment) un atome d'hydrogène ou un radical alkyle ;
X représente (notamment) NR³⁸, R³⁸ représentant notamment un atome d'hydrogène ou un radical alkyle ou aralkyle ;
n est un entier de 0 à 6 ;
Ω représente l'un des radicaux NR⁴⁶R⁴⁷ ou OR⁴⁸ dans lesquels R⁴⁶ et R⁴⁷ représentent (notamment), indépendamment, un atome d'hydrogène ou un radical alkyle, aryle, aralkyle, cycloalkyle ou cycloalkylalkyle et R⁴⁸ représente un atome d'hydrogène ou un radical alkyle.

Ces composés ne présentaient toutefois pas d'activité vis-à-vis des NOS comme les composés de l'invention.

De plus WO 98/58934 décrit des composés inhibiteurs de NOS de formule :

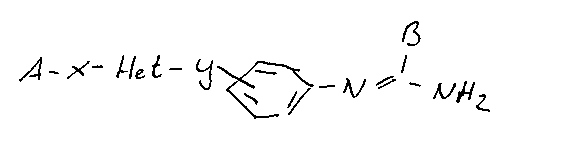

Par ailleurs, la demande WO 95/05363 a pour objet des composés inhibiteurs de NOS de formule générale **(A2)** 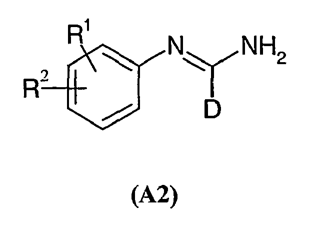 dans laquelle :
D représente phényle, pyridinyle ou un hétérocycle aromatique à 5 chaînons contenant de 1 à 4 hétéroatomes choisis parmi O, N et S, ces trois groupes étant éventuellement substitués par un ou des groupes choisis parmi (C₁-C₆)alkyle, (C₁-C₆)alkoxy, halogène, (C₁-C₆)perfluoroalkyle, ou D représente (C₁-C₆)perfluoroalkyle ;
R¹ représente hydrogène ;
R² représente -X(CH₂)ₙ,ZCONR³R⁴, -X(CH₂)ₙNHCO(CH₂)ₛNR³R⁴, -X(CH₂)ₚNR³R⁴, -X(CH₂)ₙNHCOR⁵ ou -(GH₂)_{q}NHC(NH)R⁶,
R³ et R⁴ représentent indépendamment hydrogène, (C₁-C₆)alkyle, -(CH₂)ᵣ-A, -(CH₂)ₘOA, ou -CH(CH₃)(CH₂)ₜA ;
   ou l'ensemble NR³R⁴ représente 1-indanyle, pipéronylamino, pipéridynyle, morpholinyle, pyrrolidinyle, 1,2,3,4-tétrahydroisoquinolinyle ou pipérazinyle éventuellement substitué en position 4 par (C₁-C₆)alkyle ;
R⁵ représente (C₁-C₆)alkyle, (C₁-C₆)perfluoroalkyle, -(CH₂)ᵣ-A ou -O(CH₂)_{w}A ;
A représente phényle, pyridinyle, pyrimidinyle ou un hétérocycle aromatique à 5 chaînons contenant de 1 à 4 hétéroatomes choisis parmi O, N et S, ces 4 groupes étant éventuellement substitués par un ou des groupes choisis parmi (C₁-C₆)alkyle, halogène, nitro, cyano et trifluorométhyle ;
R⁶ représente phényle, pyridinyle ou un hétérocycle aromatique à 5 chaînons contenant de 1 à 4 hétéroatomes choisis parmi O, N et S, ces trois groupes étant éventuellement substitués par un ou des groupes choisis parmi (C₁-C₆)alkyle, (C₁-C₆)alkoxy, halogène, (C₁-C₆)perfluoroalkyle, ou R⁶ représente (C₁-C₆)perfluoroalkyle ;
n et r représentent indépendamment des entiers de 0 à 6 ;
p et w représentent indépendamment des entiers de 1 à 5 ;
m représente un entier de 2 à 5 ;
q et t représentent indépendamment des entiers de 0 à 5 ;
s représente un entier de 1 à 3 ;
X représente O ou une liaison ;
Z représente O, NR⁷ ou une liaison ;
R⁷ représente hydrogène ou (C₁-C₆)alkyle ;
étant entendu que :
   (a) D, lorsque qu'il contient un hétéroatome, n'est pas relié au reste du composé de formule (**A2**) par l'hétéroatome ;
   (b) lorsque R² représente -X(CH₂)ₙZCONR³R⁴ et ni X ni Z ne représentent une liaison, alors n représente un entier de 2 à 6 ;
   (c) lorsque R² représente -X(CH₂)ₙNHCO(CH₂)ₛNR³R⁴ ou -X(CH₂)ₙNHCOR⁵ et X représente O, alors n représente un entier de 2 à 6 ;
   (d) lorsque R² représente -X(CH₂)ₚNR³R⁴ et X représente O, alors p représente un entier de 2 à 5 ;
   (e) lorsque R² représente -(CH₂)_{q}NHC(NH)R⁶, R¹ représente hydrogène, D représente phényle et R⁶ représente phényle, alors q ne représente pas 0 ;
   (f) lorsque R² représente -(CH₂)_{q}NHC(NH)R⁶, R¹ représente hydrogène, D et R⁶ représentent 2-chlorophényle, alors q ne représente pas 0 ;
   (g) lorsque R² représente -(CH₂)_{q}NHC(NH)R⁶, R¹ représente hydrogène, D et R⁶ représentent 3-pyridinyle, alors q ne représente pas 0 ; et
   (h) lorsque R² représente -(CH₂)_{q}NHC(NH)R⁶, R¹ représente hydrogène, D et R⁶ représentent 4-pyridinyle, alors q ne représente pas 0.

Toutefois, aucune activité vis-à-vis des canaux sodiques n'a été décrite pour les composés de formule générale (**A2**).

A présent, la demanderesse a mis au point une nouvelle classe de dérivés d'arylimidazoles, lesquels répondent à la formule générale **(I)** 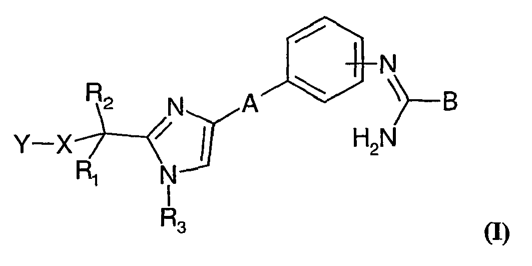 dans laquelle
R₁ représente un atome d'hydrogène ou un radical alkyle, cycloalkyle, cycloalkylalkyle, ou encore l'un des radicaux aryle ou aralkyle dont le noyau aromatique est éventuellement substitué de 1 à 3 fois (et en particulier de 1 à 2 fois) par des substituants choisis indépendamment parmi un atome halogène et un radical alkyle ou alkoxy ;
R₂ représente un atome d'hydrogène ou un radical alkyle ;
R₃ représente un atome d'hydrogène ou un radical alkyle ou aralkyle ;
X représente une liaison ou un radical alkylène linéaire ou ramifié comptant de 1 à 5 atomes de carbone ;
Y représente un atome d'hydrogène, un radical cycloalkyle, un radical NR₄R₅, OR₁₄ ou SR₁₅ ou un radical 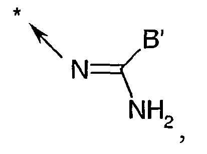 ou encore Y représente un radical aryle éventuellement substitué de 1 à 3 fois (et en particulier de 1 à 2 fois) par des substituants choisis indépendamment parmi un atome halogène et un radical alkyle ou alkoxy ;
A représente une liaison ou le radical phénylène ;
B et B' sont choisis indépendamment parmi un radical alkyle, un radical cycloalkyle, un radical NR₆R₇ ou SR₈, un radical aryle carbocyclique ou un radical aryle hétérocyclique à 5 ou 6 chaînons contenant de 1 à 4 hétéroatomes choisis parmi O, S et N (notamment les radicaux thiophène, furanne, pyrrole ou thiazole, et en particulier le radical 2-thiényle), lesdits radicaux aryle carbocyclique et hétérocyclique étant éventuellement substitués par un à trois groupes choisis indépendamment parmi les radicaux alkyle, alkényle ou alkoxy (et notamment par un radical choisi parmi les radicaux méthyle et méthoxy),
R₄ représente un atome d'hydrogène ou un radical alkyle, cycloalkyle, cycloalkylalkyle, -C(O)R₉, -C(O)OR₉, -C(O)NHR₉ ou -SO₂R₉, ou encore l'un des radicaux aryle ou aralkyle dont le noyau aromatique est éventuellement substitué de 1 à 3 fois (et en particulier de 1 à 2 fois) par des substituants choisis indépendamment parmi un atome halogène et un radical alkyle ou alkoxy, ou R₄ représente un radical *bis*-phénylalkyle, R₅ représente un atome d'hydrogène ou un radical alkyle, aryle ou aralkyle, ou encore R₄ et R₅ forment avec l'atome d'azote qui les porte un hétérocycle non aromatique de cinq à sept chaînons comptant de 1 à 2 hétéroatomes, les éléments pour compléter l'hétérocycle étant choisis indépendamment parmi un groupe composé de -CHR₁₀-, -NR₁₁-, -O- et -S- ;
R₆ et R₇ représentent indépendamment un atome d'hydrogène ou un radical alkyle, alkényle ou alkynyle,
   ou bien R₆ représente un radical nitro et R₇ représente un atome d'hydrogène,
   ou encore R₆ et R₇ forment avec l'atome d'azote qui les porte un hétérocycle non aromatique de cinq à six chaînons, les éléments pour compléter l'hétérocycle étant choisis indépendamment parmi un groupe composé de -CH₂-, -NR₁₂-, -O- et -S- ;
R₈ représente un radical alkyle linéaire ou ramifié ayant de 1 à 6 atomes de carbone éventuellement substitué de 1 à 3 fois (et en particulier de 1 à 2 fois) par un ou des substituants choisis indépendamment parmi un atome halogène et les radicaux -OH, amino, cyano et aryle ;
R₉ représente un radical alkyle, haloalkyle, cycloalkyle ou cycloalkylalkyle, ou encore l'un des radicaux aralkyle ou aryle carbocyclique ou hétérocyclique dont le noyau aromatique est éventuellement substitué de 1 à 3 fois (et en particulier de 1 à 2 fois) par des substituants choisis indépendamment parmi un atome halogène et un radical alkyle ou alkoxy ;
R₁₀ représente un atome d'hydrogène ou un radical alkyle ou aryle éventuellement substitué de 1 à 3 fois (et en particulier de 1 à 2 fois) par des substituants choisis indépendamment parmi un atome halogène et un radical alkyle ou alkoxy (R₁₀ étant de préférence choisi parmi un atome d'hydrogène ou un radical méthyle ou phényle),
R₁₁ représente un atome d'hydrogène, un radical alkyle, un radical cycloalkyle, un radical cycloalkylakyle, un radical -C(O)R₁₃, un radical -C(O)OR₁₃, un radical -SO₂R₁₃, un radical -C(O)NHR₁₃, ou encore l'un des radicaux aryle ou aralkyle dont le noyau aromatique est éventuellement substitué de 1 à 3 fois (et en particulier de 1 à 2 fois) par des substituants choisis indépendamment parmi un atome halogène et un radical alkyle ou alkoxy ;
R₁₂ représente un atome d'hydrogène ou un radical alkyle ;
R₁₃ représente un radical alkyle, un radical haloalkyle ou encore l'un des radicaux aralkyle ou aryle carbocyclique ou hétérocyclique dont le noyau aromatique est éventuellement substitué de 1 à 3 fois (et en particulier de 1 à 2 fois) par des substituants choisis indépendamment parmi un atome halogène et un radical alkyle ou alkoxy ;
R₁₄ représente un radical alkyle, le radical phényle ou un radical aralkyle ; et enfin
R₁₅ représente un radical alkyle, le radical phényle ou un radical aralkyle;
ou sont des sels pharmaceutiquement acceptables de composés de formule générale **(I).**

Lesdits composés de formule générale **(I)** ou leurs sels pharmaceutiquement acceptables peuvent être utilisés pour préparer un médicament destiné à inhiber les NOS et à moduler les canaux sodiques.

En particulier, les composés de formule générale **(I)** ou les sels pharmaceutiquement acceptables de composés de formule générale **(I)** seront des composés de formule générale **(I')** 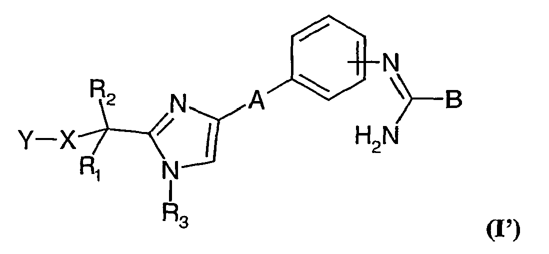 dans laquelle
R₁ représente un atome d'hydrogène ou un radical alkyle, cycloalkyle, cycloalkylalkyle, ou encore l'un des radicaux aryle ou aralkyle dont le noyau aromatique est éventuellement substitué de 1 à 3 fois (et en particulier de 1 à 2 fois) par des substituants choisis indépendamment parmi un atome halogène et un radical alkyle ou alkoxy ;
R₂ représente un atome d'hydrogène ou un radical alkyle ;
R₃ représente un atome d'hydrogène ou un radical alkyle ou aralkyle ;
X représente une liaison ou un radical alkylène linéaire ou ramifié comptant de 1 à 5 atomes de carbone ;
Y représente un atome d'hydrogène, un radical cycloalkyle, un radical NR₄R₅ ou un radical 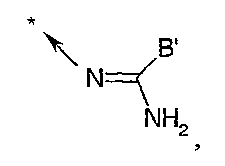 ou encore Y représente un radical aryle éventuellement substitué de 1 à 3 fois (et en particulier de 1 à 2 fois) par des substituants choisis indépendamment parmi un atome halogène et un radical alkyle ou alkoxy ;
A représente une liaison ou le radical phénylène ;
B et B' sont choisis indépendamment parmi un radical alkyle, un radical cycloalkyle, un radical NR₆R₇ ou SR₈, un radical aryle carbocyclique ou un radical aryle hétérocyclique à 5 ou 6 chaînons contenant de 1 à 4 hétéroatomes choisis parmi O, S et N (notamment les radicaux thiophène, furanne, pyrrole ou thiazole, et en particulier le radical 2-thiényle), lesdits radicaux aryle carbocyclique et hétérocyclique étant éventuellement substitués par un à trois groupes choisis indépendamment parmi les radicaux alkyle, alkényle ou alkoxy (et notamment par un radical choisi parmi les radicaux méthyle et méthoxy),
R₄ représente un atome d'hydrogène ou un radical alkyle, cycloalkyle, cycloalkylalkyle, -C(O)R₉, -C(O)OR₉, -C(O)NHR₉ ou -SO₂R₉, ou encore l'un des radicaux aryle ou aralkyle dont le noyau aromatique est éventuellement substitué de 1 à 3 fois (et en particulier de 1 à 2 fois) par des substituants choisis indépendamment parmi un atome halogène et un radical alkyle ou alkoxy,
R₅ représente un atome d'hydrogène ou un radical alkyle, aryle ou aralkyle, ou encore R₄ et R₅ forment avec l'atome d'azote qui les porte un hétérocycle non aromatique de cinq à sept chaînons comptant de 1 à 2 hétéroatomes, les éléments pour compléter l'hétérocycle étant choisis indépendamment parmi un groupe composé de -CHR₁₀-, -NR₁₁-, -O- et -S- ;
R₆ et R₇ représentent indépendamment un atome d'hydrogène ou un radical alkyle, alkényle ou alkynyle,
   ou bien R₆ représente un radical nitro et R₇ représente un atome d'hydrogène,
   ou encore R₆ et R₇ forment avec l'atome d'azote qui les porte un hétérocycle non aromatique de cinq à six chaînons, les éléments pour compléter l'hétérocycle étant choisis indépendamment parmi un groupe composé de -CH₂-, -NR₁₂-, -O- et -S- ;
R₈ représente un radical alkyle linéaire ou ramifié ayant de 1 à 6 atomes de carbone éventuellement substitué de 1 à 3 fois (et en particulier de 1 à 2 fois) par un ou des substituants choisis indépendamment parmi un atome halogène et les radicaux -OH, amino, cyano et aryle ;
R₉ représente un radical alkyle, haloalkyle, cycloalkyle ou cycloalkylalkyle, ou encore l'un des radicaux aralkyle ou aryle carbocyclique ou hétérocyclique dont le noyau aromatique est éventuellement substitué de 1 à 3 fois (et en particulier de 1 à 2 fois) par des substituants choisis indépendamment parmi un atome halogène et un radical alkyle ou alkoxy ;
R₁₀ représente un atome d'hydrogène ou un radical alkyle ou aryle éventuellement substitué de 1 à 3 fois (et en particulier de 1 à 2 fois) par des substituants choisis indépendamment parmi un atome halogène et un radical alkyle ou alkoxy (R₁₀ étant de préférence choisi parmi un atome d'hydrogène ou un radical méthyle ou phényle),
R₁₁ représente un atome d'hydrogène, un radical alkyle, un radical cycloalkyle, un radical cycloalkylalkyle, un radical -C(O)R₁₃, un radical -C(O)OR₁₃, un radical -SO₂R₁₃, un radical -C(O)NHR₁₃, ou encore l'un des radicaux aryle ou aralkyle dont le noyau aromatique est éventuellement substitué de 1 à 3 fois (et en particulier de 1 à 2 fois) par des substituants choisis indépendamment parmi un atome halogène et un radical alkyle ou alkoxy ;
R₁₂ représente un atome d'hydrogène ou un radical alkyle ; et enfin
R₁₃ représente un radical alkyle, un radical haloalkyle ou encore l'un des radicaux aralkyle ou aryle carbocyclique ou hétérocyclique dont le noyau aromatique est éventuellement substitué de 1 à 3 fois (et en particulier de 1 à 2 fois) par des substituants choisis indépendamment parmi un atome halogène et un radical alkyle ou alkoxy ;
ou seront des sels pharmaceutiquement acceptables de composés de formule générale **(I').**

Par alkyle, lorsqu'il n'est pas donné plus de précision, on entend un radical alkyle linéaire ou ramifié comptant de 1 à 12 atomes de carbone, de préférence de 1 à 8 atomes de carbone et encore plus préférentiellement de 1 à 6 atomes de carbone. Par cycloalkyle, lorsqu'il n'est pas donné plus de précision, on entend un radical cycloalkyle comptant de 3 à 7 atomes de carbone. Par alkoxy, lorsqu'il n'est pas donné plus de précision, on entend un radical alkoxy dont la chaîne carbonée est linéaire ou ramifiée et compte de 1 à 6 atomes de carbone. Par alkényle, lorsqu'il n'est pas donné plus de précision, on entend un radical hydrocarboné linéaire ou ramifié comptant de 2 à 6 atomes de carbone et au moins une double liaison. Par alkynyle, lorsqu'il n'est pas donné plus de précision, on entend un radical hydrocarboné linéaire ou ramifié comptant de 2 à 6 atomes de carbone et au moins une triple liaison. Par haloalkyle, on entend un radical alkyle dont au moins l'un des atomes d'hydrogène (et éventuellement tous) est remplacé par un atome halogène. Par haloalkoxy, on entend un radical alkoxy dont au moins l'un des atomes d'hydrogène (et éventuellement tous) est remplacé par un atome halogène. Par aryle carbocyclique ou hétérocyclique, lorsqu'il n'est pas donné plus de précision, on entend un système carbocyclique ou hétérocyclique comprenant de un à trois cycles condensés dont l'un au moins est un cycle aromatique et tous sont des cycles de 5 à 7 chaînons, un système étant dit hétérocyclique lorsque l'un au moins des cycles qui le composent comporte un ou des hétéroatomes (O, N ou S). Par aryle, lorsqu'il n'est pas donné plus de précision, on entend un radical aryle carbocyclique. Enfin, par atome halogène, on entend un atome choisi parmi les atomes de fluor, de chlore, de brome et d'iode.

Par les radicaux cycloalkylalkyle, aralkyle, alkoxycarbonyle, haloalkoxycarbonyle ou aralkoxycarbonyle, on entend respectivement les radicaux cycloalkylalkyle, aralkyle, alkoxycarbonyle, haloalkoxycarbonyle ou aralkoxycarbonyle dont les radicaux alkyle, alkoxy, haloalkyle, cycloalkyle et aryle qui les composent ont les significations indiquées précédemment.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par cycloalkyle, on entend notamment les radicaux cyclopropyle, cyclobutyle, cyclopentyle et cyclohexyle. Par alkoxy, on entend de préférence les radicaux méthoxy, éthoxy, propoxy, isopropoxy, isobutoxy, sec-butoxy et tert-butoxy et plus préférentiellement les radicaux méthoxy et éthoxy. Par haloalkyle, on entend notamment le radical trifluorométhyle. Par haloalkoxy, on entend notamment le radical trifluorométhoxy. Par aryle carbocyclique, on entend notamment les radicaux phényle, naphtyle et phénantryle, de préférence les radicaux phényle et naphtyle et plus préférentiellement le radical phényle. Par aryle hétérocyclique, on entend notamment les radicaux pyrrolyle, furannyle, thiényle, pyridyle, pyrimidinyle, triazinyle, imidazolyle, oxazolyle, thiazolyle, indolyle et quinolyle. Par aralkyle, on entend notamment un radical phénalkyle, et de préférence le radical benzyle.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Pour d'autres exemples de sels pharmaceutiquement acceptables, on peut se référer à "Salt selection for basic drugs", Int. J. Pharm. (1986), 33, 201-217.

Par ailleurs, par convention, lorsqu'il émane d'une structure chimique une flèche dirigée vers une étoile (*), ladite flèche indique le point d'attache. Par exemple : 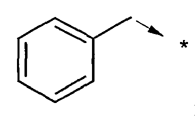 représente le radical benzyle.

D'une façon générale, le cas dans lequel A représentera une liaison sera préféré. De même, on préférera d'un façon générale les composés dans lesquels R₃ représente un atome d'hydrogène ou un radical méthyle ou benzyle (et en particulier les composés dans lesquels R₃ représente un atome d'hydrogène).

De préférence par ailleurs, les composés de formule générale **(I)** selon l'invention seront tels qu'ils comportent au moins l'une des caractéristiques suivantes :
◆ X représente une liaison ou un radical alkylène linéaire ou ramifié comptant de 1 à 5 atomes de carbone (et de préférence de 1 à 3 atomes de carbone) et Y représente un radical NR₄R₅ ;
◆ X représente une liaison ou un radical alkylène linéaire ou ramifié comptant de 1 à 5 atomes de carbone (et de préférence de 1 à 3 atomes de carbone) et Y représente un radical 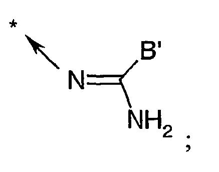
◆ X représente une liaison ou un radical alkylène linéaire ou ramifié comptant de 1 à 5 atomes de carbone (et de préférence de 1 à 3 atomes de carbone) et Y représente un radical cycloalkyle ou un radical aryle éventuellement substitué de 1 à 3 fois (et en particulier de 1 à 2 fois) par des substituants choisis indépendamment parmi un atome halogène et un radical alkyle ou alkoxy ;
◆ X représente une liaison et Y représente un atome d'hydrogène tandis que l'un au moins de R₁ et R₂ représente un radical choisi parmi les radicaux alkyle, cycloalkyle ou cycloalkylakyle.

Selon l'une des variantes préférées, à savoir lorsque X représente une liaison ou un radical alkylène linéaire ou ramifié comptant de 1 à 5 atomes de carbone (et de préférence de 1 à 3 atomes de carbone et encore plus préférentiellement de 1 à 2 atomes de carbone) et Y représente un radical NR₄R₅, on préférera en outre que les composés de formule générale **(I)** selon l'invention soient tels qu'ils comportent au moins l'une des caractéristiques suivantes :
◆ R₄ représente un radical alkyle, cycloalkyle, cycloalkylalkyle ou encore l'un des radicaux aryle ou aralkyle dont le noyau aromatique est éventuellement substitué de 1 à 3 fois par des substituants choisis indépendamment parmi un atome halogène et un radical alkyle ou alkoxy (R₄ représentant plus préférentiellement encore un radical choisi parmi les radicaux alkyle et cycloalkyle et encore plus préférentiellement un radical cycloalkyle), et R₅ représente un atome d'hydrogène
ou un radical alkyle (et plus préférentiellement un atome d'hydrogène ou le radical méthyle) ;
◆ R₄ représente un radical -C(O)R₉, -C(O)OR₉, -C(O)NHR₉ ou -SO₂R₉ (et tout particulièrement un radical-C(O)OR₉) et R₅ représente un atome d'hydrogène ou un radical méthyle ou éthyle (et de préférence un atome d'hydrogène ou le radical méthyle).

Selon une autre variante préférée, à savoir lorsque X représente une liaison ou un radical alkylène linéaire ou ramifié comptant de 1 à 5 atomes de carbone (et de préférence de 1 à 3 atomes de carbone) et Y représente un radical 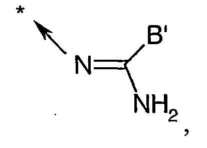 on préférera en outre que les composés de formule générale **(I)** selon l'invention soient tels qu'ils comportent au moins l'une des caractéristiques suivantes :
◆ X représente un liaison ou un radical-CH₂- ou -(CH₂)₂- ;
◆ R₁ et R₂ représentent des atomes d'hydrogène.

Toujours selon l'une des variantes préférées, à savoir lorsque X représente une liaison ou un radical alkylène linéaire ou ramifié comptant de 1 à 5 atomes de carbone (et de préférence de 1 à 3 atomes de carbone) et Y représente un radical cycloalkyle ou un radical aryle éventuellement substitué de 1 à 3 fois (et en particulier de 1 à 2 fois) par des substituants choisis indépendamment parmi un atome halogène et un radical alkyle ou alkoxy, on préférera en outre que les composés de formule générale **(I)** selon l'invention soient tels qu'ils comportent au moins l'une des caractéristiques suivantes :
◆ Y est un radical cyclohexyle ;
◆ Y est un radical phényle éventuellement substitué de 1 à 3 fois (et en particulier de 1 à 2 fois) par des substituants choisis indépendamment parmi un atome halogène et un radical alkyle ou alkoxy (et de préférence parmi un atome halogène et un radical méthyle ou méthoxy) ;
◆ R₁ et R₂ représentant des atomes d'hydrogène.

Selon encore une autre variante préférée, à savoir lorsque X représente une liaison et Y représente un atome d'hydrogène tandis que l'un au moins de R₁ et R₂ représente un radical choisi parmi les radicaux alkyle, cycloalkyle ou cycloalkylakyle, on préférera en outre que les composés de formule générale **(I)** selon l'invention soient tels qu'ils comportent au moins l'une des caractéristiques suivantes :
◆ l'un au moins de R₁ et R₂ représente un radical alkyle comptant au moins 4 atomes de carbone tandis que l'autre représente un atome d'hydrogène ;
◆ R₁ et R₂ représentent tous deux des radicaux alkyle comptant au moins 3 atomes de carbone chacun ;
◆ R₁ représente un radical cycloalkyle ou cycloalkylalkyle et R₂ représente alors de préférence un atome d'hydrogène ou un radical méthyle (et plus préférentiellement un atome d'hydrogène).

D'une manière générale, on préférera les composés de formule générale **(I)** dans lesquels B représente un radical cycloalkyle (notamment le radical cyclopropyle), un radical aryle carbocyclique (notamment le radical phényle), un radical aryle hétérocyclique à 5 chaînons contenant de 1 à 2 hétéroatomes choisis parmi O, S et N (notamment les radicaux thiophène, furanne, pyrrole ou thiazole, et en particulier le radical 2-thiényle) ou encore le radical NH-NO₂. On préférera particulièrement les composés de formule générale **(I)** dans lesquels B représente soit un radical aryle hétérocyclique à 5 chaînons contenant de 1 à 2 hétéroatomes choisis parmi O, S et N (notamment les radicaux thiophène, furanne, pyrrole ou thiazole, et en particulier le radical 2-thiényle), soit le radical NH-NO₂. On préférera encore plus particulièrement les composés de formule générale **(I)** dans lesquels B représente le radical 2-thiényle ou le radical NH-NO₂. Les mêmes préférences sont applicables *mutatis mutandis* à B' lorsque ce radical est présent dans les composés de formule générale **(I).**

Egalement de façon générale, lorsque R₄ et R₅ forment avec l'atome d'azote qui les porte un hétérocycle non aromatique de cinq à sept chaînons comptant de 1 à 2 hétéroatomes, les éléments pour compléter l'hétérocycle étant choisis indépendamment parmi un groupe composé de -CHR₁₀-, -NR₁₁-, -O- et -S-, l'hétérocycle formé (qui est éventuellement substitué par des radicaux R₁₀ et R₁₁) est de préférence choisi parmi le groupe composé des cycles pyrrolidine, pipéridine, pipérazine, morpholine, thiomorpholine, azépine et homopipérazine, et plus préférentiellement parmi le groupe composé des cycles pyrrolidine, pipéridine, pipérazine, morpholine et thiomorpholine.

Par ailleurs, R₁₃ représentera de préférence un radical alkyle ou un radical haloalkyle.

De plus, R₁₅ sera de préférence le radical phényle, tout comme R₁₄.

En particulier, l'invention concerne les composés de formule générale **(I)** suivants, décrits plus loin en tant qu'exemples (parfois sous forme de sels) :
- 2-[4-(4-{[(1Z)-amino(thién-2-yl)méthylène]-amino}phényl)-1*H*-imidazol-2-yl]éthylcarbamate de butyle ;
- 2-[4-(3-{[(1E)-amino(thién-2-yl)méthylène]-amino}phényl)-1H-imidazol-2-yl]éthylcarbamate de butyle ;
- 2-[4-(4'-{[(1Z)-amino(thién-2-yl)méthylène]amino}-1,1'-biphényl-4-yl)-1H-imidazol-2-yl]éthylcarbamate de butyle ;
- N'-(4-{2-[(cyclohexylamino)méthyl]-1H-imidazol-4-yl}phényl)thiophène-2-carboximidamide ;
- *N*'-(4-{2-[2-(cyclohexylamino)éthyl]-1H-imidazol-4-yl}phényl)thiophène-2-carboximidamide ;
- *N*'-(3-{2-[(cyclohexylamino)méthyl]-1*H*-imidazol-4-yl}phényl)thiophène-2-carboximidamide ;
- *N*-[4-(2-{[cyclohexyl(méthyl)amino]méthyl}-1*H*-imidazol-4-yl)phényl]thiophène-2-carboximidamide ;
- *N*'-(4-{2-[(dibenzylamino)méthyl]-1*H*-imidazol-4-yl}phényl)thiophène-2-carboximidamide ;
- *N*-(4-{2-[(benzylamino)méthyl]-1*H*-imidazol-4-yl}phényl)thiophène-2-carboximidamide ;
- *N*'-{3-[2-(aminométhyl)-1H-imidazol-4-yl]phényl}thiophène-2-carboximidamide ;
- *N*'-{3-[2-({[(1*E*)-amino(thién-2-yl)méthylène]-amino}méthyl)-1*H*-imidazol-4-yl]phényl}thiophène-2-carboximidamide ;
- *N*'-{4-[2-({[(1*E*)-amino(thién-2-yl)méthylène]-amino}méthyl)-1*H*-imidazol-4-yl]phényl} thiophène-2-carboximidamide ;
- *N*-{3-[2-(2-cyclohexyléthyl)-1*H*-imidazol-4-yl]phényl}thiophène-2-carboximidamide ;
- *N'*- {3-[2-(1-pentylhexyl)-1*H*-imidazol-4-yl]phényl}thiophène-2-carboximidamide ;
- *N*'-{4-[2-(2-cyclohexyléthyl)-1*H*-imidazol-4-yl]phényl}thiophène-2-carboximidamide ;
- *N*'-{3-[2-(cyclohexylméthyl)-1*H*-imidazol-4-yl]phényl}thiophène-2-carboximidamide ;
- *N*'-{3-[2-(3-cyclohexylpropyl)-1*H*-imidazol-4-yl]phényl}thiophène-2-carboximidamide ;
- *N*'-[3-(2-hexyl-1*H*-imidazol-4-yl)phényl]thiophène-2-carboximidamide ;
- *N*-{4-[2-(2-cyclohexyléthyl)-1*H*-imidazol-4-yl]phényl}-*N*"-nitroguanidine ;
- *N*'-(4-{2-[(cycloheptylamino)méthyl]-1*H*-imidazol-4-yl}phényl)thiophène-2-carboximidamide;
- *N*'-(4-{2-[(méthylamino)méthyl]-1*H*-imidazol-4-yl}phényl)thiophène-2-carboximidamide;
- *N*-(4-{2-[(cyclobutylamino)méthyl]-1H-imidazol-4-yl}phényl)thiophène-2-carboximidamide;
- *N*'-[4-(2-{[(2,2-diphényléthyl)amino]méthyl}-1H-imidazol-4-yl)phényl]thiophène-2-carboximidamide;
- *N'-* {3-[2-(2-{ [(1 E)-amino(thién-2-yl)méthylène] amino} éthyl)-1H-imidazol-4-yl]phényl} thiophène-2-carboximidamide;
- *N'*-(3-{2-[(phénylthio)méthyl]-1H-imidazol-4-yl}phényl)thiophène-2-carboximidamide;
- *N*'-(4-{2-[(phénylthio)méthyl]-1H-imidazol-4-yl}phényl)thiophène-2-carboximidamide;
- *N*'- {3-[2-(4-isobutylbenzyl)-1*H*-imidazol-4-yl]phényl} thiophène-2-carboximidamide;
et les sels de ces derniers.

L'invention concerne aussi, à titre de médicaments, les composés de formule générale **(I)** telle que définie précédemment ou les sels pharmaceutiquement acceptables de tels composés. Elle concerne de même les compositions pharmaceutiques contenant, à titre de principe actif, les composés de formule générale **(I)** telle que définie précédemment ou les sels pharmaceutiquement acceptables de tels composés, avec un ou des excipients phannaceutiquement acceptables.

De plus, l'invention a encore pour objet l'utilisation de composés de formule générale **(I)** telle que définie précédemment ou des sels pharmaceutiquement acceptables de tels composés pour préparer un médicament destiné à traiter ou prévenir un désordre choisi / une maladie choisie parmi les désordres suivants / maladies suivantes : les douleurs, la sclérose en plaques, les troubles du système nerveux central ou périphérique, les troubles cardiovasculaires, les troubles du muscle squelettique et des jonctions neuromusculaires, les maladies inflammatoires, les pertes d'audition d'origine traumatique, acoustique ou toxique et des acouphènes, les complications liées aux maladies auto-immunes et virales et les maladies neurologiques associées à des intoxications, à des traitements ou à des désordres d'origine génétique. De préférence, l'invention concernera l'utilisation de composés de formule générale **(I)** telle que définie précédemment ou des sels pharmaceutiquement acceptables de tels composés pour préparer un médicament destiné à traiter un désordre choisi / une maladie choisie parmi les désordres suivants / maladies suivantes : les douleurs, les troubles du système nerveux central ou périphérique. De façon plus particulière, l'invention concernera l'utilisation de composés de formule générale **(I)** telle que définie précédemment ou des sels pharmaceutiquement acceptables de tels composés pour préparer un médicament destiné à traiter ou prévenir la douleur, en particulier la douleur d'origine neuropathique.

L'invention concerne également les méthodes de traitement des maladies susmentionnées comprenant l'administration au patient à soigner d'une dose thérapeutiquement efficace d'un composé de formule générale **(I).**

Pour les médicaments, les compositions pharmaceutiques, les utilisations pour préparer des médicaments ou les utilisations thérapeutiques, les préférences indiquées pour les composés de formule générale **(I)** s'appliquent *mutatis mutandis.*

Dans certains cas, les composés de formule générale **(I)** selon la présente invention peuvent comporter des atomes de carbone asymétriques. Par conséquent, ces composés auront deux formes énantiomères possibles, c'est-à-dire les configurations "R" et "S". La présente invention inclut les deux formes énantiomères des composés de formule générale **(I)** et toutes combinaisons de ces formes, y compris les mélanges racémiques "RS". Dans un souci de simplicité, lorsqu'aucune configuration spécifique n'est indiquée dans les formules de structure ou les noms des composés, il faut comprendre que les deux formes énantiomères et leurs mélanges sont représentés.

Les compositions pharmaceutiques contenant un composé de l'invention peuvent être sous forme d'un solide, par exemple des poudres, des granules, des comprimés, des gélules, des liposomes, des suppositoires ou des patchs. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, par voie orale, par voie parentérale, par injection intramusculaire, par injection sous-cutanée, par injection intra-veineuse, etc.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A titre indicatif, la dose d'administration envisagée pour un médicament selon l'invention est comprise entre 0,1 mg à 10 g suivant le type de composé actif utilisé.

Conformément à l'invention, on peut préparer les composés de formule générale **(I)** par les procédés décrits ci-dessous.

### PREPARATION DES COMPOSES DE L'INVENTION :

### Préparation des composés de formule générale (I) :

Les composés de formule générale **(I)** peuvent être préparés par exemple à partir des intermédiaires de formules générales **(I)_{P}, (I)_{AP}, (I)_{AD}, (I)_{D}, (II)₁, (II)_{1A}, (III)₁, (III)_{1A}, (IV), (IV'), (V)**, **(VI), (VI'), (VII)** et **(VII')** selon les procédures exposées ci-après.

### CAS n° 1 : R₃ représente un atome d'hydrogène :

### Voie n°1 :

Y représente H ou un radical cycloalkyle ou aryle éventuellement substitué, ou encore Y représente un radical OR₁₄, SR₁₅ ou NR₄R₅ dans lequel R₄ représente -(C)OR₉, -C(O)OR₉, -C(O)NHR₉ ou -SO₂R₉, ou dans lequel R₄ répresente un radical alkyle, cycloalkyle, cycloalkylalkyle ou aryle *bis*-phénylalkyle ou aralkyle éventuellement substitué et R₅ ne représente pas H, ou encore dans lequel R₄ et R₅ forment avec l'atome d'azote qui les porte un hétérocycle :

Lorsque Y représente un atome d'hydrogène, un radical cycloalkyle, un radical aryle éventuellement substitué ou un radical OR₁₄, SR₁₅ ou NR₄R₅ dans lequel R₄ représente un radical -C(O)R₉, -C(O)OR₉, -C(O)NHR₉ ou -SO₂R₉, ou dans lequel R₄ représente un radical alkyle, cycloalkyle, cycloalkylalkyle ou *bis*-phénylalkyle ou un radical aryle ou aralkyle éventuellement substitué sur le noyau aromatique et R₅ ne représente pas un atome d'hydrogène ou encore dans lequel R₄ et R₅ forment avec l'atome d'azote qui les porte un hétérocycle éventuellement substitué, les composés de formule générale **(I)** correspondants, ci-après dénommés composés de formule générale **(I)₁,** peuvent être préparés, schéma 1, à partir des intermédiaires de formule générale **(II)₁** dans laquelle X, R₁, R₂ et A ont la même signification que dans la formule générale **(I),** W représente un groupe NO₂ ou N₃ et Y représente un atome d'hydrogène, un radical cycloalkyle ou un radical NR₄R₅ dans lequel R₄ et R₅ ont les mêmes significations que ci-dessus. Lesdits intermédiaires de formule générale **(II)₁** sont soumis, dans un solvant polaire protique tel que l'éthanol (éventuellement en mélange avec du dichlorométhane), à une hydrogénation catalysée par du palladium sur charbon (ou à toute autre réaction appropriée) pour donner les intermédiaires de formule générale **(III)₁.** Les composés de formule générale **(I)₁** sont alors obtenus par réaction, dans un solvant tel que l'isopropanol, des intermédiaires de formule générale **(III)₁** soit avec l'un des composés de formules générales **(IV), (V), (VI)** ou **(VII)** (une déprotection en milieu acide du composé obtenu de façon intermédiaire étant encore nécessaire dans le cas de la réaction avec le composé de formule générale **(VII)),** soit avec le benzoylisothiocyanate suivi d'un halogénoalkyle de formule générale R₈-Hal.

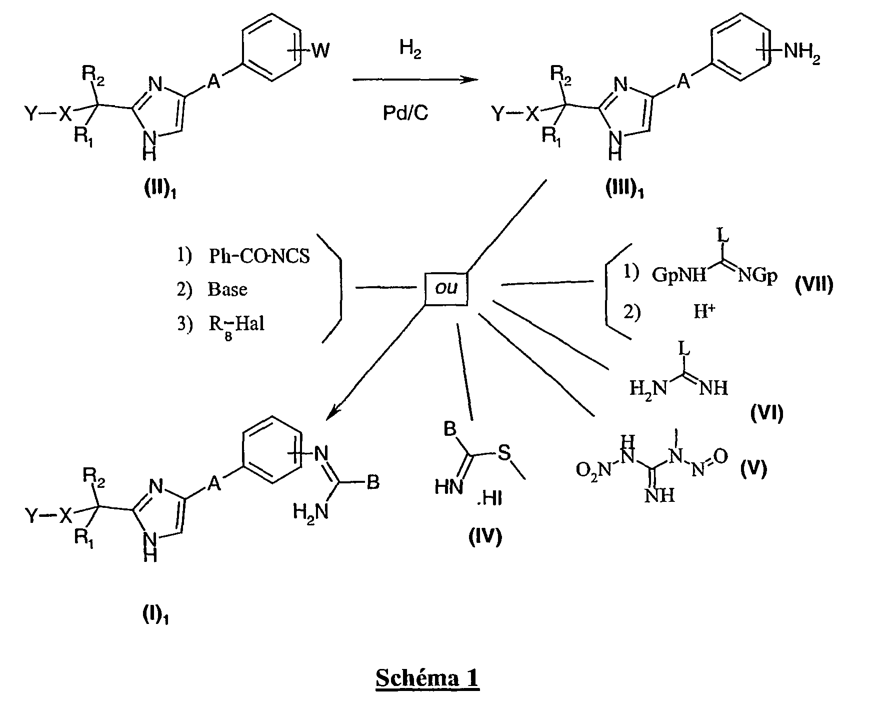

Ainsi, dans le cas particulier où B représente un radical alkyle, un radical cycloalkyle, un radical NR₆R₇ dans lequel R₆ et R₇ forment avec l'atome d'azote qui les porte un hétérocycle non aromatique de cinq à six chaînons, un radical aryle carbocyclique ou un radical aryle hétérocyclique, l'on pourra effectuer la conversion du composé de formule générale **(III)₁** avec le composé de formule générale **(IV)** comme représenté dans le schéma 1.

Dans le cas particulier où B représente NHNO₂, le composé de formule générale **(III)₁** sera condensé avec le composé de formule (**V**) comme représenté dans le schéma 1.

Dans le cas particulier où B représente un radical NR₆R₇ dans lequel R₆ représente un atome d'hydrogène ou un radical alkyle, alkényle ou alkynyle et R₇ représente un atome d'hydrogène ou un radical alkyle, le composé de formule générale **(III)₁** pourra être condensé avec le composé de formule générale **(VI)** dans lequel L représente, par exemple, un cycle pyrazole ou encore avec le composé de formule générale (**VII**) dans lesquels, par exemple L représente un cycle pyrazole et Gp le groupement Boc *(*Tetrahedron Lett. (1993) 34 (21), 3389-3392) ou bien L représente le groupement -N-SO₂-CF₃ et Gp le groupement Boc (J. Org. Chem. (1998) 63, 3804-3805). Dans le cas où l'on utilise un composé de formule générale **(VII),** la déprotection de la fonction guanidine est ensuite effectuée, par exemple, en présence d'un acide fort tel que par exemple l'acide trifluoroacétique, pour conduire au composé de formule générale **(I)₁.**

Enfin, dans le cas particulier où B représente un radical SR₈, les thiourées de formule générale **(I)₁** pourront être préparées en 3 étapes. La réaction du benzoylisothiocyanate sur l'aniline de formule générale **(III)₁** dans un solvant tel que, par exemple, l'acétone, conduit à l'intermédiaire benzoyl-thiourée qui est ensuite classiquement hydrolysé par chauffage en milieu basique. La thiourée ainsi obtenue est ensuite alkylée en utilisant, par exemple, un dérivé halogéné de formule générale R₈-Hal, par chauffage dans un solvant inerte, pour conduire au composé de formule générale **(I)₁.**

### Voie n° 2 :

Y représente H ou un radical cycloalkyle un radical aryle éventuellement substitué ou encore un radical NR₄R₅ dans lequel R₄ ne représente pas -C(O)R₉, -C(O)OR₉, -C(O)NHR₉ ou -SO₂R₉, ou dans lequel R₄ représente un radical alkyle, cycloalkyle, cycloalkylalkyle ou *bis-* phénylalkyle ou encore aryle ou aralkyle éventuellement substitué et R₅ représente H :

Lorsque Y représente un radical NR₄R₅ dans lequel R₄ ne représente pas un radical -C(O)R₉, -C(O)OR₉, -C(O)NHR₉ ou -SO₂R₉ ou un radical aryle éventuellement substitué, ou dans lequel R₄ représente un radical alkyle, cycloalkyle, cycloalkylalkyle ou *bis*-phénylalkyle ou un radical aryle ou aralkyle éventuellement substitué sur le noyau aromatique et R₅ représente un atome d'hydrogène, les composés de formule générale **(I)** correspondants, ci-après dénommés composés de formule générale **(I)₂,** peuvent être préparés, schéma 2, à partir des composés de formule générale **(I)_{P}** dans laquelle X, R₁, R₂, R₅, A et B ont la même signification que dans la formule générale **(I)** et Gp représente un groupe protecteur classique d'amine (tel qu'un groupe *tert*-butoxycarbonyle). Lesdits composés de formule générale **(I)_{P}** sont déprotégés pour donner les composés de formule générale **(I)_{D},** la réaction étant effectuée dans des conditions classiques pour l'homme du métier (cf. *Protective groups in organic synthesis,* 2nd ed., (John Wiley & Sons Inc., 1991)) ; cette déprotection s'effectuera ainsi par exemple en milieu acide (notamment en utilisant de l'acide chlorhydrique, la réaction pouvant s'effectuer dans un solvant comme l'acétate d'éthyle). Les composés de formule générale **(I)_{D}** sont ensuite alkylés selon des techniques connues de l'homme du métier pour donner les composés de formule générale **(I)₂** ; par exemple, dans le cas où R₄ représente un radical alkyle ou cycloalkylalkyle, l'on fera réagir les composés de formule générale **(I)_{D}** avec des halogénures de formule générale R₄ Hal dans laquelle Hal est un atome halogène, ou encore, dans le cas où R₄ représente un radical cycloalkyle ou un radical de formule générale R-CH₂- dans laquelle R représente un radical alkyle, aralkyle ou *bis*-phénylalkyle, l'on effectuera une réaction de condensation des composés de formule générale **(I)_{D}** avec les cétones appropriées ou l'aldéhyde de formule générale R-CHO en présence d'un agent réducteur comme le triacétoxyborohydrure de sodium ou le borohydrure de sodium dans un alcool aliphatique inférieur comme le méthanol et éventuellement en présence de tamis moléculaires, cette réaction étant de préférence effectuée à température ambiante.

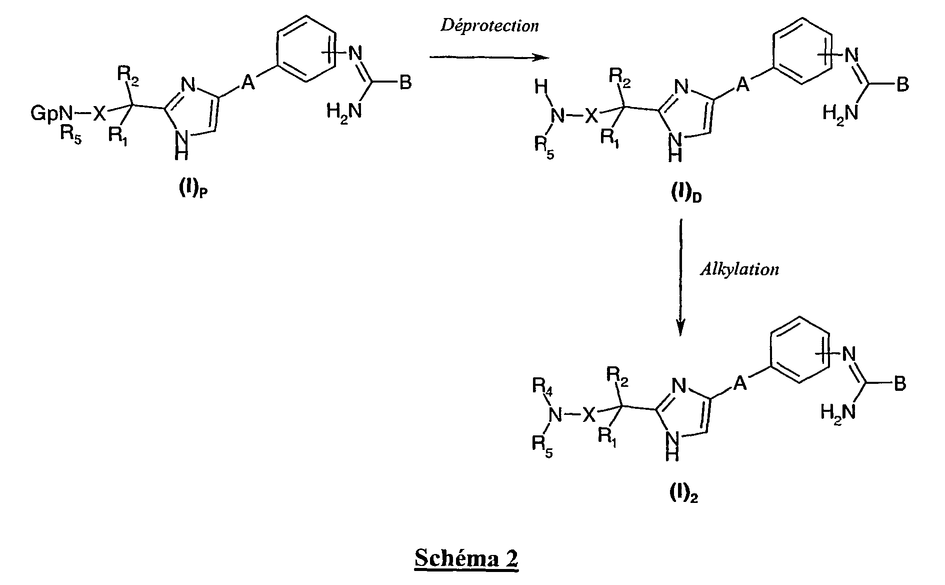

### Voie n° 3 :

Y représente un radical du type amidine :

Lorsque Y représente un radical du type 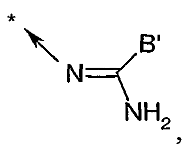 les composés de formule générale **(I)** correspondants, ci-après dénommés composés de formule générale **(I)₃,** peuvent être préparés, schéma 3, à partir des composés de formule générale **(I)₁** dans laquelle Y représente un radical NH₂. Les composés de formule générale **(I)₂** sont alors obtenus par réaction, dans un solvant tel que l'isopropanol, des composés de formule générale **(I)₁** dans laquelle X, R₁, R₂, A et B ont la même signification que dans la formule générale **(I)** et Y représente un radical NH₂ soit avec l'un des composés de formules générales **(IV'), (V), (VI')** ou **(VII')** (une déprotection en milieu acide du composé obtenu de façon intermédiaire étant encore nécessaire dans le cas de la réaction avec le composé de formule générale **(VII')),** soit avec le benzoylisothiocyanate suivi d'un halogénoalkyle de formule générale R₈-Hal (cf. CAS n° 1, voie n° 1).

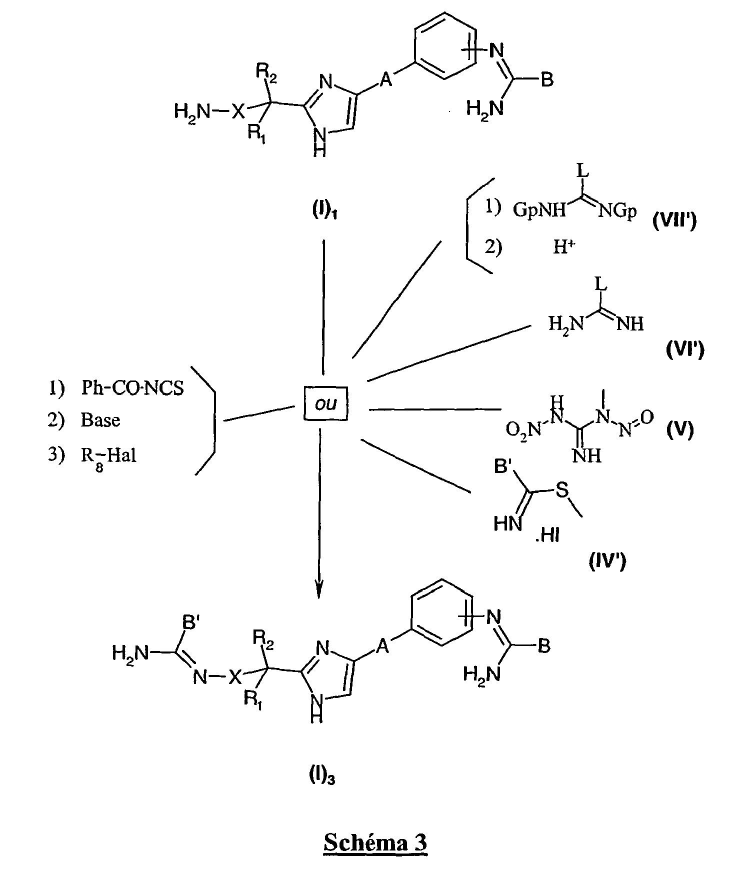

### CAS n° 2 : R₃ ne représente pas un atome d'hydrogène :

### Voie n°1 :

Y représente H ou un radical cycloalkyle ou un radical éventuellement substitué, ou encore Y représente un radical OR₁₄, SR₁₅ ou NR₄R₅ dans lequel R₄ représente - C(O)R₉, -C(O)OR₉, -C(O)NHR₉ ou -SO₂R₉, ou dans lequel R₄ représente un radical alkyle, cycloalkyle, cycloalkylalkyle ou *bis*-phénylalkyle ou aryle ou aralkyle éventuellement substitué et R₅ ne représente pas H, ou encore dans lequel R₄ et R₅ forment avec l'atome d'azote qui les porte un hétérocycle :

Lorsque R₃ représente un radical alkyle ou aralkyle et Y représente un atome d'hydrogène, un radical cycloalkyle, un radical aryle éventuellement substitué ou un radical OR₁₄, SR₁₅ ou NR₄R₅ dans lequel R₄ représente un radical -C(O)R₉, -C(O)OR₉, -C(O)NHR₉ ou -SO₂R₉, ou dans lequel R₄ représente un radical alkyle, cycloalkyle, cycloalkylalkyle ou *bis*-phénylalkyle ou un radical aryle ou aralkyle éventuellement substitué sur le noyau aromatique et R₅ ne représente pas un atome d'hydrogène ou encore dans lequel R₄ et R₅ forment avec l'atome d'azote qui les porte un hétérocycle éventuellement substitué, l'on pourra simplement ajouter une étape à la voie de synthèse n° 1 du CAS 1. L'atome d'azote en position 1 du noyau imidazole sera alkylé avant de procéder à l'hydrogénation catalysée, puis les étapes habituelles de la voie n° 1 du CAS *n°* 1 seront utilisées pour donner les composés de formule générale **(I)_{1A},** autrement dit les composés de formule générale **(I)₁** dans laquelle R₃ représente un radical alkyle ou aralkyle et X, Y, R₁, R₂, A et B ont les mêmes significations que dans la formule générale **(I)₁.** Cette méthode de synthèse est résumée dans le schéma 1*bis* ci-après.

Les composés de formule générale **(I)_{1A},** peuvent être préparés, schéma 1 *bis,* à partir des intermédiaires de formule générale **(II)₁** dans laquelle X, R₁, R₂ et A ont la même signification que dans la formule générale **(I),** W représente un groupe NO₂ ou N₃, Y représente un atome d'hydrogène, un radical cycloalkyle, un radical aryle éventuellement substitué, un radical OR₁₄ ou SR₁₅, ou un radical NR₄R₅ dans lequel R₄ représente un radical -C(O)R₉, -C(O)OR₉, -C(O)NHR₉, -SO₂R₉ ou aryle éventuellement substitué, ou dans lequel R₄ représente un radical alkyle, cycloalkyle, cycloalkylalkyle ou *bis*-phénylalkyle ou encore un radical aralkyle éventuellement substitué et R₅ ne représente pas un atome d'hydrogène. Lesdits intermédiaires de formule générale **(II)₁** sont d'abord alkylés selon des techniques connues de l'homme du métier, par exemple au moyen d'un dérivé halogéné de formule générale R₃Hal dans laquelle Hal est un atome halogène. Les intermédiaires de formule générale **(II)_{1A}** sont ensuite soumis, dans un solvant polaire protique tel que l'éthanol (éventuellement en mélange avec du dichlorométhane), à une hydrogénation catalysée par du palladium sur charbon pour donner les intermédiaires de formule générale **(III)_{1A}.** Les composés de formule générale **(I)_{1A}** sont alors obtenus par réaction, dans un solvant tel que l'isopropanol, des intermédiaires de formule générale **(III)_{1A}** soit avec les composés de formules générales **(IV), (V), (VI)** ou **(VII)** (une déprotection en milieu acide du composé obtenu de façon intermédiaire étant encore nécessaire dans le cas de la réaction avec le composé de formule générale **(VII)),** soit avec le benzoylisothiocyanate puis un halogénoalkyle de formule générale R₈-Hal (cf. CAS n° 1, voie n °1).

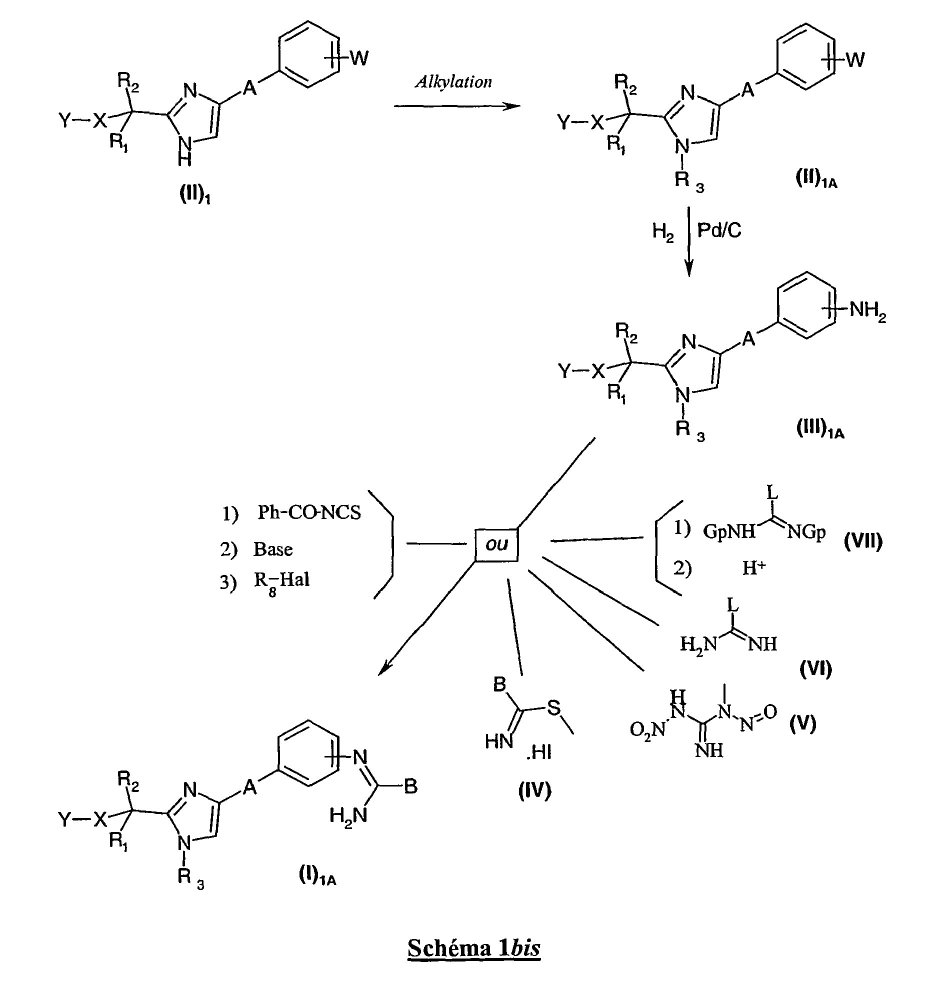

### Voie n°2 :

Y représente H ou un radical cycloalkyle ou aryle éventuellement substitué ou encore un radical NR₄R₅ dans lequel R₄ ne représente pas -C(O)R₉, -C(O)OR₉, -C(O)NHR₉ ou -SO₂R₉, ou dans lequel R₄ représente un radical alkyle, cycloalkyle, cycloalkylalkyle ou *bis*-phénylalkyle ou encore aryle ou aralkyle éventuellement substitué, et R₅ représente H :

Lorsque R₃ représente un radical alkyle ou aralkyle et Y représente un radical NR₄R₅ dans lequel R₄ ne représente pas un radical -C(O)R₉, -C(O)OR₉, -C(O)NHR₉ ou - SO₂R₉ ou dans lequel R₄ représente un radical alkyle, cycloalkyle, cycloalkylalkyle ou *bis*-phénylalkyle ou un radical aryle ou aralkyle éventuellement substitué sur le noyau aromatique et R₅ représente un atome d'hydrogène, l'on pourra simplement ajouter une étape à la voie de synthèse n° 2 du CAS n° 1. L'atome d'azote en position 1 du noyau imidazole sera alkylé tandis que la fonction amine de la chaîne latérale en position 2 du noyau imidazole sera protégée, puis les étapes habituelles de la voie n° 2 du CAS n° 1 seront utilisées pour donner les composés de formule générale **(I)_{2A},** autrement dit les composés de formule générale **(I)₂** dans laquelle R₃ représente un radical alkyle ou aralkyle et A, B, X, R₁, R₂, R₄ et R₅ ont les mêmes significations que dans la formule générale **(I)₂.** Cette méthode de synthèse est résumée dans le schéma 2*bis* ci-après.

Les composés de formule générale **(I)_{P}** sont d'abord alkylés selon des techniques connues de l'homme du métier, par exemple au moyen d'un dérivé halogéné de formule générale R₃Hal dans laquelle Hal est un atome halogène. Les composés de formule générale **(I)_{AP}** obtenus sont ensuite déprotégés pour donner les composés de formule générale **(I)_{AD},** la réaction étant effectuée dans des conditions classiques pour l'homme du métier (cf. Protective groups in organic synthesis, 2nd ed., (John Wiley & Sons Inc., 1991)) ; cette déprotection s'effectuera ainsi par exemple en milieu acide (notamment en utilisant de l'acide chlorhydrique, la réaction pouvant s'effectuer dans un solvant comme l'acétate d'éthyle). Les composés de formule générale **(I)_{AD}** sont ensuite alkylés selon des techniques connues de l'homme du métier pour donner les composés de formule générale **(I)_{2A} ;** par exemple, dans le cas où R₄ représente un radical alkyle ou cycloalkylalkyle, l'on fera réagir les composés de formule générale **(I)_{AD}** avec des halogénures de formule générale R₄Hal dans laquelle Hal est un atome halogène, ou encore, dans le cas où R₄ représente un radical cycloalkyle ou un radical de formule générale R-CH₂- dans laquelle R représente un radical alkyle, aralkyle ou *bis*-phénylalkyle, l'on effectuera une réaction de condensation des composés de formule générale **(I)_{AD}** avec les cycloalkylcétones appropriées ou l'aldéhyde de formule générale R-CHO en présence d'un agent réducteur comme le triacétoxyborohydrure de sodium ou le borohydrure de sodium dans un alcool aliphatique inférieur comme le méthanol et éventuellement en présence de tamis moléculaires, cette réaction étant de préférence effectuée à température ambiante.

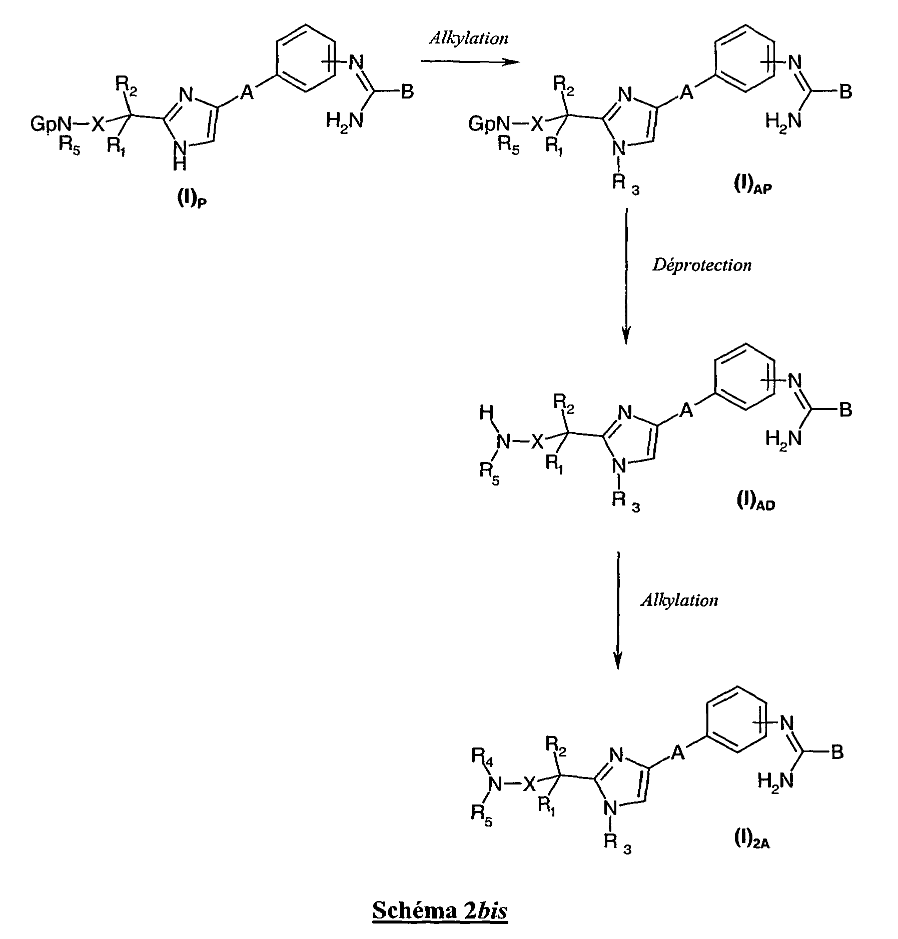

### Voie n° 3 :

Y représente un radical du type amidine :

Lorsque Y représente un radical du type 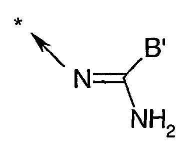 et R₃ représente un radical alkyle ou aralkyle, les composés de formule générale **(I)** correspondants, ci-après dénommés composés de formule générale **(I)_{3A},** peuvent être préparés selon une méthode analogue à celle décrite dans la voie de synthèse n° 3 du CAS n° 1, le composé de formule générale **(I)₁** dans laquelle Y représente NH₂ et R₃ représente un atome d'hydrogène étant simplement remplacé par le composé de formule générale **(I)_{1A}** dans laquelle Y représente NH₂ et R₃ représente un radical alkyle ou aralkyle. Cette méthode de synthèse est résumée dans le schéma 3*bis* ci-après.

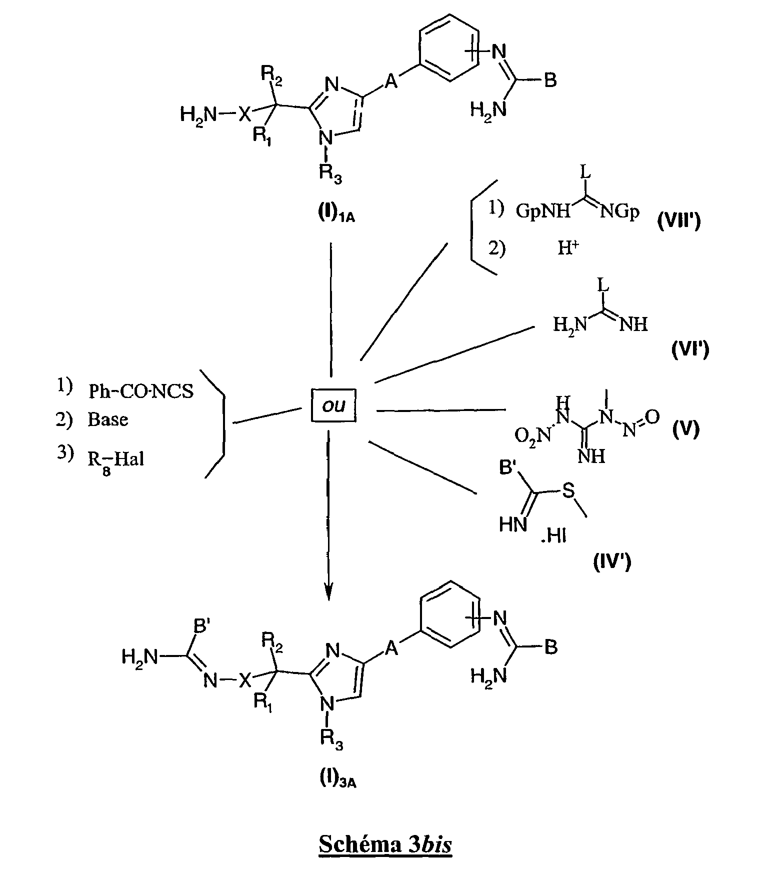

### Préparation de certains intermédiaires de synthèse non commerciaux :

### Préparation des composés de formule générale (I)_{P}

Ces composés sont en fait également des composés de formule générale **(I)** dans lesquels Y représente un groupe NR₄R₅ dans lequel R₄ représente un groupe alkoxycarbonyle particulier (tel qu'un groupe *tert*-butoxycarbonyle). Ils peuvent donc être préparés selon la procédure décrite pour les composés de formule générale **(I)** ci-dessus (cf. voie 1).

### Préparation des composés de formule générale (II)₁

Les composés de formule générale **(II)₁** sont obtenus par réaction de cyclocondensation de l'acide de formule générale **(VIII)₁** avec l'α-halogénocétone de formule générale **(IX)₁.** Par exemple, schéma 4, du carbonate de césium est ajouté à l'acide de formule générale **(VIII)₁.** L'intermédiaire obtenu est condensé avec une α-halogénocétone de formule générale **(IX)₁.** puis un large excès d'acétate d'ammonium (par exemple 15 ou 20 équivalents par équivalent d'acide de formule générale **(VIII)₁)** est ajouté. Cette réaction s'effectue de préférence dans un mélange de xylènes et en chauffant (on peut aussi, le cas échéant, éliminer simultanément l'eau formée au cours de la réaction).

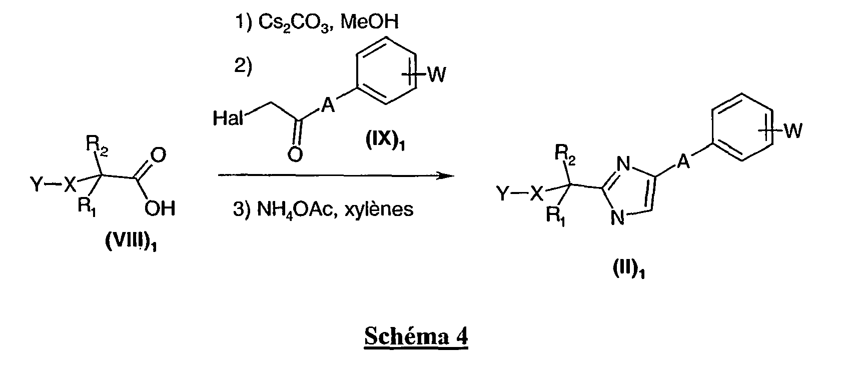

### Préparation des composés de formule générale (IV) ou (IV')

Les composés de formule générale **(IV)** dans laquelle B représente un radical alkyle, un radical aryle carbocyclique ou un radical aryle hétérocyclique peuvent être obtenus, par exemple, schéma 5, par réaction des thioamides de formule générale **(IV).1** avec de l'iodure de méthyle dans un solvant tel que l'acétone. La synthèse est identique pour les composés de formule générale **(IV') ((IV').1** et B' remplaçant respectivement **(IV).1** et B dans le schéma 5)

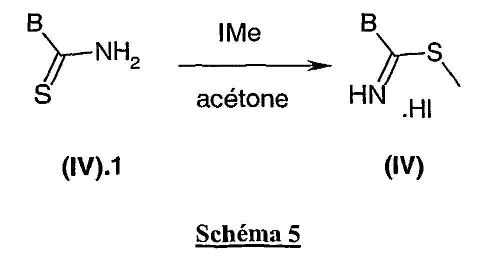

### Préparation du composé de formule (V)

La préparation de ces composés peut être effectuée selon des méthodes courantes pour l'homme du métier comme par exemple celle décrite dans la publication suivante : J. Amer. Chem. Soc. (1947), 69, 3028-3030).

### Préparation de certains composés de formule générale (VIII)₁.

Les composés de formule générale **(VIII)₁** dans lesquels R₄ représente un radical -C(O)OR₉ et X, R₁, R₂, R₅ et R₉ ont la même signification que dans la formule générale **(I)** sont préparés, schéma 6, par réaction, dans des conditions basiques (créées par exemple par ajout d'hydroxyde de sodium et d'eau ou en présence d'une base organique telle que la triéthylamine), de l'aminoacide de formule générale **(X)₁** avec le dérivé halogéné de formule générale R₉-OC(O)-Hal dans laquelle Hal représente un atome halogène. Une fois la réaction terminée, le milieu est acidifié (par exemple par ajout d'acide chlorhydrique) pour donner l'aminoacide de formule générale **(VIII)₁.**

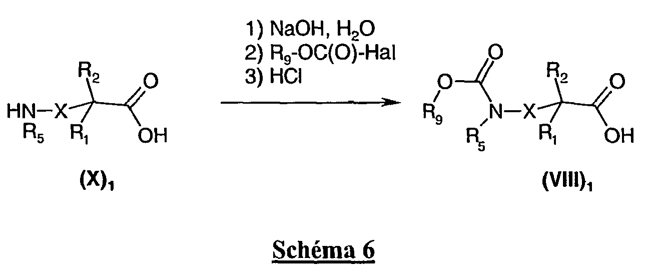

Les composés de formule générale **(VIII)₁** dans lesquels R₄ représente un radical -C(O)R₉ et X, R₁, R₂ et R₅ ont la même signification que dans la formule générale **(I)** sont préparés, schéma 7, par condensation des aminoacides de formule générale **(X)₁** avec les acides carboxyliques (ou les chlorures d'acide correspondants) de formule générale R₉-COOH dans les conditions classiques de la synthèse peptidique (M. Bodanszky et A. Bodanszky, The Practice of Peptide Synthesis, 145 (Springer-Verlag, 1984)) dans un solvant polaire comme le tétrahydrofuranne, le dichlorométhane ou le diméthylformamide en présence d'un réactif de couplage tels que le dicyclohexylcarbodiimide (DCC), le 1,1'-carbonyldiimidazole (CDI) *(*J. Med. Chem. (1992), 35 (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)).

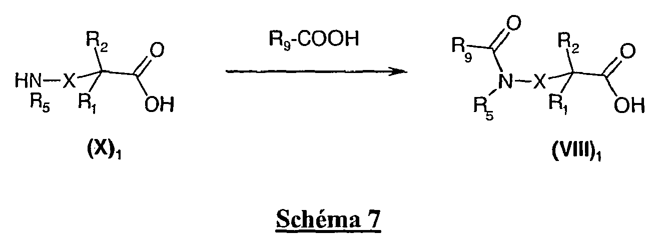

Les composés de formule générale **(VIII)₁** dans lesquels R₄ représente un radical -C(O)NHR₉ et X, R₁, R₂ et R₅ ont la même signification que dans la formule générale **(I)** sont préparés, schéma 8, par réaction des aminoacides de formule générale **(X)₁** avec les isocyanates de formule générale R₉-NCO, la réaction pouvant être effectuée à température ambiante dans un solvant tel que le chloroforme.

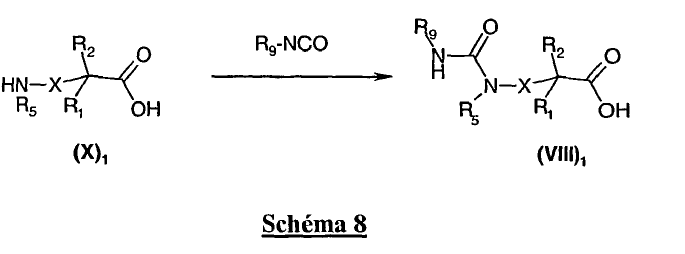

Les composés de formule générale **(VIII)₁** dans lesquels R₄ représente un radical -C(O)NHR₉ et X, R₁, R₂ et R₅ ont la même signification que dans la formule générale **(I)** sont préparés, schéma 9, par réaction des aminoacides de formule générale **(X)₁** avec les sulfochlorures de formule générale R₉-SO₂Cl dans des conditions classiques, la réaction pouvant par exemple être effectuée à température ambiante dans un solvant tel que le diméthylformamide en présence d'une base telle que la triéthylamine.

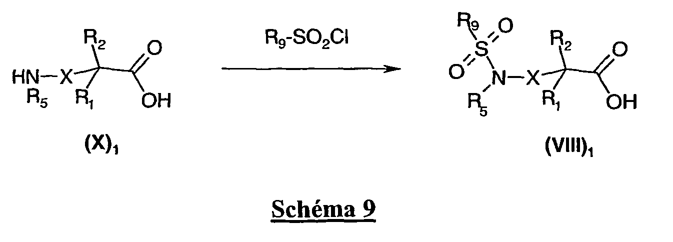

### Préparation des composés de formule générale (IX)₁

Les composés de formule générale **(IX)₁,** dans laquelle Hal représente un atome halogène (par exemple un atome de chlore ou de brome), W représente un groupe NO₂ ou N₃ et A a la même signification que dans la formule générale **(I),** sont préparés, schéma 10, par réaction de la cétone de formule générale **(XI)₁** avec un agent halogénant. Par exemple, dans le cas particulier d'une bromation, la réaction pourra être effectuée avec un agent de bromation tel que CuBr₂ (J. Org. Chem. (1964), 29, 3459), du brome (J. Het. Chem. (1988), 25, 337), du N-bromosuccinimide *(*J. Amer. Chem. Soc. (1980), 102, 2838) en présence d'acide acétique dans un solvant comme l'acétate d'éthyle ou le dichlorométhane, HBr ou Br₂ dans de l'éther, de l'éthanol ou de l'acide acétique (Biorg. Med. Chem. Lett. (1996), 6(3), 253-258; J. Med. Chem. (1988), 31(10), 1910-1918 ; J. Am. Chem. Soc. (1999), 121, 24) ou encore à l'aide d'une résine de bromation (J. Macromol. Sci. Chem. (1977), A11, (3) 507-514).

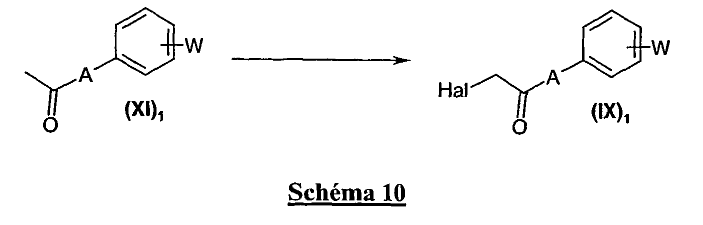

### Préparation des autres intermédiaires

La préparation des autres intermédiaires non commerciaux est décrite dans la littérature ou à la portée de l'homme du métier par le biais de méthodes de synthèse classiques.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES:

### Méthode employée pour la mesure du temps de rétention (t.r.) et du pic moléculaire (MH+).

Les composés sont caractérisés par leur temps de rétention (t.r.), exprimé en minutes, déterminé par chromatographie liquide (CL), et leur pic moléculaire (MH+) déterminé par spectrométrie de masse (SM), un spectromètre de masse simple quadripôle (Micromass, modèle Platform) équipé d'une source électrospray est utilisé avec une résolution de 0,8 da à 50% de vallée.
Pour les exemples ci-après, les conditions d'élution correspondant aux résultats indiqués sont les suivantes : passage d'un mélange acétonitrile-eau-acide trifluoroacétique 50-950-0,2 (A) à un mélange acétonitrile-eau 950-50 (B) par un gradient linéaire sur une période de 8,5 minutes, puis élution avec le mélange B pur pendant 10,5 minutes.

### Exemple 1 : chlorhydrate de 2-[4-(4-{[(1Z)-amino(thién-2-yl)méthylène]-amino}phényl)-1H-imidazol-2-yl]éthylcarbamate de butyle :

### 1.1) 2-[4-(4-azidophényl)-1H-imidazol-2-yl]éthylcarbamate de butyle:

Un mélange contenant de la *N*-(butoxycarbonyl)-β-alanine (3 g ; 15,1 mmol) et du carbonate de césium (2,43 g ; 7,55 mmol) dans 50 ml de méthanol anhydre est agité pendant une heure. Ce mélange est évaporé à sec puis dilué avec 60 ml de diméthylformamide. Du bromure de 4-azidophénacyle est ajouté (3,64 g ; 15,1 mmol) puis le mélange résultant agité durant 2 h. Le solvant est évaporé à l'aide d'une pompe à palettes. 80 ml d'acétate d'éthyle sont ajoutés et le bromure de césium est filtré sur fritté. Après évaporation du filtrat, 200 ml de xylènes sont ajoutés. De l'acétate d'ammonium (23 g ; 0,3 mol) est alors ajouté et le mélange chauffé au reflux pendant 1 h 30 avant d'être versé dans de l'eau glacée à laquelle on ajoute 80 ml d'acétate d'éthyle. Après décantation, la phase organique est lavée avec une solution saturée en chlorure de sodium. La phase organique est ensuite séchée sur sulfate de magnésium et le solvant évaporé. L'huile obtenue est purifiée sur une colonne de silice (éluant : acétate d'éthyle-heptane / 8-2). Le produit attendu est récupéré sous forme d'une huile noire avec un rendement de 65%.

### 1.2) 2-[4-(4-aminophényl)-1H-imidazol-2-yl]éthylcarbamate :

De l'intermédiaire 1.1 (3 g ; 9,15 mmol) est dissous dans 50 ml d'éthanol en présence de palladium sur charbon (environ 10% massique). Ce mélange est hydrogéné sous deux bars pendant 18 h. Le mélange réactionnel est ensuite filtré sur filtre Millipore^{®} puis rincé à l'éthanol. Après évaporation du solvant, on obtient une mousse de couleur brun clair avec un rendement de 88%.
RMN ¹H (δ ppm, DMSO): 0,84 (t, 3H); 1,27-1,29 (m, 2H); 1,45-1,48 (m, 2H) ; 3,05 (m, 2H) ; 3,42 (m, 2H) ; 3,90 (m, 2H) ; 5,5-6,2 (m large, 1H) ; 6,67-6,69 (d, 2H) ; 7,34 (s large, 1H) ; 7,47-7,49 (d, 2H) ; 7,70 (s, 1H) ; 14,33 (s large, 2H).
MH+ = 303,2.

### 1.3) Thiophéne-2-carbimidothioate de méthyle:

De l'iodure de méthyle (66 g ; 0,46 mol) est ajouté goutte à goutte à 0 °C à une solution de thiophène-2-carbothioamide (50 g ; 0,33 mol) dans 500 ml d'acétone. Après l'addition, l'agitation est maintenue pendant deux heures à 23 °C. Le précipité formé est filtré sur fritté et lavé par deux fois avec 100 ml d'acétone avant d'être séché sous vide (dans une cloche). On obtient une poudre jaune avec un rendement de 97%.
RMN ¹H (δ ppm, DMSO) : 2,8 (s, 3H) ; 7,42 (m, 1H); 8,125 (d, 1H) ; 8,27 (d, 1H) ; 10-12 (m large, 1H).

### 1.4) 2-[4-(4-{[(1Z)-amino(thién-2-yl)méthylène]amino}phényl)-1H-imidazol-2-yl]éthylcarbamate de butyle :

De l'intermédiaire 1.2 (2,5 g; 8,2 mmol) est mis en suspension dans 30 ml de 2-propanol en présence de l'intermédiaire 1.3 (1,2 éq.). Le mélange réactionnel est maintenu à 50 °C pendant 18 h avant d'être concentré à sec. Le résidu est repris dans 50 ml d'acétate d'éthyle et 50 ml d'une solution saturée en carbonate de sodium. Le mélange est agité pendant 30 minutes avant d'être décanté. La phase organique est ensuite lavée avec une solution saturée en chlorure de sodium puis séchée sur sulfate de sodium. Les solvants sont évaporés et la mousse obtenue est purifiée sur une colonne de silice (éluant : CH₂Cl₇-MeOH / 97-3 à 90-10). On obtient une poudre de couleur jaune clair avec un rendement de 57%. Point de fusion : 146,2 °C.
MH+ = 412,2.

### 1.5) Chlorhydrate de 2"-[4-(4-{[(1Z)-amino(thién-2-yl)méthylène]amino}phényl)-1H-imidazol-2-yl]éthylcarbamate de butyle:

L'intermédiaire 1.4 (1 g ; 2,43 mmol) est dissous dans de l'éthanol (20 ml). De l'acide chlorhydrique 1*N* en solution dans de l'éther (9,7 ml ; 9,7 mmol). On agite le mélange pendant une heure. Après concentration à sec, le résidu est repris dans de l'éther (15 ml) et le mélange est agité 15 min. Après filtration sur fritté et lavage avec de l'éther du solide récupéré, ce dernier est séché sous vide (cloche). Le produit attendu est obtenu sous forme d'un solide de couleur crème avec un rendement de 100%. Point de fusion : > 260°C.
NM+ = 412,2.

*Les composés des exemples 2 et 3 sont préparés selon un mode opératoire analogue à celui décrit pour le composé de l'exemple 1.*

### Exemple 2 : chlorhydrate de 2-[4-(3-{[(1E)-amino(thién-2-yl)méthylène]-amino}phényl)-1H-imidazol-2-yl]éthylcarbamate de butyle :

Point de fusion : 210-212 °C.
MH+ = 412,2.

### Exemple 3 : chlorhydrate de 2-[4-(4'-{[(1Z)-amino(thién-2-yl)méthylène]amino}-1,1'-biphényl-4-yl)-1H-imidazol-2-yl]éthylcarbamate de butyle :

Point de fusion : 97-98 °C.
MH+ = 488,2.

### Exemple 4 : chlorhydrate de N'-(4-{2-[(cyclohexylamino)méthyl]-1H-imidazol-4-yl}phényl)thiophène-2-carboximidamide :

### 4.1) [4-(4-azidophényl)-1H-imidazol-2-yl]méthylcarbamate de tert-butyle :

Un mélange contenant de la *N*-(*tert*-butoxycarbonyl)-glycine (5 g ; 28,5 mmol) et du carbonate de césium (4,6 g ; 14,2 mmol) dans 30 ml de méthanol anhydre est agité pendant une heure. Ce mélange est évaporé à sec puis dilué avec 60 ml de diméthylformamide. Du bromure de 4-azidophénacyle (6,84 g ; 28,5 mmol) est ajouté puis le mélange résultant agité durant 2 h. Le solvant est évaporé à l'aide d'une pompe à palettes. 80 ml d'acétate d'éthyle sont ajoutés et le bromure de césium est filtré sur fritté. Après évaporation du filtrat, 200 ml de xylènes sont ajoutés, puis de l'acétate d'ammonium (44 g ; 0,57 mol), et le mélange est chauffé au reflux pendant 1 h 30 avant d'être versé dans de l'eau glacée à laquelle à laquelle ont été ajoutés 80 ml d'acétate d'éthyle. Après décantation, la phase organique est neutralisée avec une solution saturée en bicarbonate de sodium suivi d'une filtration sur filtre à microfibres de verre (GF/A, Whatman) La phase organique est lavée avec une solution saturée en chlorure de sodium puis séchée sur sulfate de sodium et le solvant évaporé. L'huile noire obtenue est purifiée sur une colonne de silice (éluant : acétate d'éthyle-heptane / 6-4 à 3-7). On obtient une poudre de couleur brune qui, après lavage dans l'éther isopropylique, conduit à une poudre de couleur brun clair avec un rendement de 48%.
RMN ¹H (δ ppm, DMSO): 1,39 (s, 9H); 4,16 (m, 2H); 7,06 (d, 2H) ; 7,24 (s large, 1H) ; 7,48 (s, 1H) ; 7,77 (d, 2H) ; 11,8 (s large, 1H).

### 4.2) [4-(4-aminophényl)-1H-imidazol-2-yl]méthylcarbamate de tert-butyle :

De l'intermédiaire 4.1 (4,3 g ; 13,6 mmol) est dissous dans 50 ml d'un mélange éthanol-CH₂Cl₂ 2-1 en présence de palladium sur charbon (environ 10% massique). Ce mélange est hydrogéné sous une pression de 2 bars d'hydrogène pendant 24 h. Le mélange réactionnel est ensuite filtré sur filtre à microfibres de verre (GF/A, Whatman) puis rincé à l'éthanol. Après évaporation du solvant, on agite le résidu dans de l'éther. Le mélange est ensuite filtré sur fritté puis lavé à l'éther et à l'isopentane. Une poudre de couleur jaune pâle est obtenue avec un rendement de 100%.
RMN ¹H (δ ppm, DMSO): 1,39 (s, 9H); 4,17 (m, 2H) ; 6,54 (d, 2H); 7,16-7,19 (s large, 2H); 7,35 (d, 2H).

### 4.3) [4-(4-{[(1E)-amino(thién-2-yl)méthylène]amino}phényl)-1H-imidazol-2-yl]méthylcarbamate de tert-butyle :

De l'intermédiaire 4.2 (3,9 g ; 13,6 mol) est mise en suspension dans 30 ml de propanol-2 en présence de l'intermédiaire 1.3. Le mélange est chauffé à une température de 60 °C pendant 48 h. Après concentration à sec du mélange, le résidu est repris dans 50 ml d'acétate d'éthyle et 50 ml d'une solution saturée en hydrogénocarbonate de sodium. Le mélange est agité pendant 30 minutes avant d'être décanté. La phase organique récupérée est lavée avec une solution saturée en chlorure de sodium puis séchée sur sulfate de sodium. Les solvants sont évaporés et la mousse obtenue est purifiée sur une colonne de silice (éluant : CH₂Cl₂-EtOH / 98-2 à 90-10). On obtient une huile de couleur jaune clair qui cristallise dans l'éther. Après filtration sur fritté et lavage à l'éther, une poudre de couleur jaune pâle est obtenue avec un rendement de 59%.
RMN ¹H (δ ppm, DMSO) : 1,39 (s, 9H) ; 4,17 (m, 2H) ; 6,4 (m, 2H) ; 6,82 (m, 2H) ; 7,07-7,36 (m, 3H) ; 7,59-7,73 (m, 4H) ; 11,7 (s large, 1H).

### 4.4) chlorhydrate de N'-{4-[2-(aminométhyl)-1H-imidazol-4-yl]phényl}thiophène-2-carboximidamide :

L'intermédiaire 4.3 (3,1 g ; 7,79 mmol) est mis en suspension dans 20 ml d'acétate d'éthyle. De l'acide chlorhydrique de concentration 4,4 M dans de l'acétate d'éthyle (80 ml ; 0,35 mol) est ajouté et le mélange obtenu agité à 22 °C pendant 18 h. Après concentration à sec, le résidu est repris dans de l'éther avant d'être reconcentré à sec. Après agitation dans de l'isopentane puis filtration sur fritté et rinçage du solide à l'isopentane, une poudre de couleur crème est obtenue avec un rendement de 95%. Point de fusion : > 260 °C.
RMN ¹H (δ ppm, DMSO): 4,47 (s, 2H); 7,37-7,39 (m, 1H) ; 7,59 (m, 2H) ; 8,05 (m, 2H) ; 8,18 (m, 3H); 9,08 (m large, 3H); 9,94 (s large, 1H) ; 11,6-11,8 (s large, 1H).

### 4.5) N-(4-{2-[(cyclohexylamino)méthyl]-1H-imidazol-4-yl}phényl)thiophène-2-carboximidanzide :

De l'intermédiaire 4.4 (800 mg ; 1,96 mmol) est mis en suspension dans 30 ml de méthanol en présence de triéthylamine (0,83 ml ; 5,88 mmol). De la cyclohexanone (0,25 ml ; 2,35 mmol) est alors ajoutée puis le mélange est agité pendant trois heures à 23 °C. Après addition de triacétoxyborohydrure de sodium (500 mg ; 2,35 mmol), le mélange est de nouveau laissé agiter à 23 °C durant deux heures. Une solution saturée en hydrogénosulfate de potassium est alors ajoutée, puis de l'eau pour solubiliser le précipité qui s'est formé. Enfin, une solution saturée en bicarbonate de sodium est ajoutée au mélange obtenu avant quintuple extraction à l'acétate d'éthyle. La phase organique est ensuite séchée sur sulfate de sodium. Les solvants sont évaporés et la mousse obtenue est purifiée sur une colonne de silice (éluant : CH₂Cl₂-EtOH-NH₄OH / 92,5-5,5-2 à 90-7,5-2,5). On obtient une huile de couleur jaune clair qui cristallise dans l'éther. Après filtration sur fritté et lavage à l'éther du solide, une poudre de couleur jaune pâle est obtenue avec un rendement de 64%.
RMN ¹H (δ ppm, DMSO) : 1,02-1,22 (s, 5H) ; 1,52-1,84 (m, 5H); 2,37-2,42 (m, 1H); 3,75 (s, 2H) ; 6,41 (m, 2H); 6,82 (m, 2H) ; 7,08 (m, 1H); 7,36 (m, 1H); 7,59-7,74 (m, 4H) ; 11,7 (s large, 1H).
MH+ = 380,2.

### 4.6) chlorhydrate de N-(4-{2-[(cyclohexylamino)méthyl]-1H-imidazol-4-yl}phényl)thiophène-2-carboximidamide :

Ce composé est obtenu à partir de l'intermédiaire 4.5 selon un mode opératoire analogue à celui de l'étape 1.5 de l'exemple 1. Point de fusion : 234-235 °C.
MH+ = 394,2.

*Les composés des exemples 5 à 9 sont préparés selon un mode opératoire analogue à celui décrit pour le composé de l'exemple 4.*

### Exemple 5 : chlorhydrate de N'-(4-{2-[2-(cyclohexylamino)éthyl]-1H-imidazol-4-yl}phényl)thiophène-2-carboximidamide :

Point de fusion: 185-186 °C.
MH+ = 394,2.

### Exemple 6 : chlorhydrate de N'-(3-{2-[(cyclohexylamino)méthyl]-1H-imidazol-4-yl}phényl)thiophène-2-carboximidamide :

Point de fusion : > 225°C.
MH+ = 380,2.

### Exemple 7 : chlorhydrate de N'-[4-(2-{[cyclohexyl(méthyl)amino]méthyl}-1H-imidazol-4-yl)phényl]thiophène-2-carboximidamide :

Point de fusion : 240-241 °C.

### Exemple 8 : chlorhydrate de N'-(4-{2-[(dibenzylamino)méthyl]-1H-imidazol-4-yl}phényl)thiophène-2-carboximidamide :

Point de fusion : 150-151 °C.
MH+ = 478,2.

### Exemple 9 : chlorhydrate de N'-(4-{2-[(benzylamino)méthyl]-1H-imidazol-4-yl}phényl)thiophène-2-carboximidamide :

Point de fusion : 228-229 °C.
MH+ = 388,1.

### Exemple 10 : chlorhydrate de N'-{3-[2-(aminométhyl)-1H-imidazol-4-yl]phényl}thiophène-2-carboximidamide :

### 10.1) [4-(3-nitrophényl)-1H-imidazol-2-yl]méthylcarbamate de tert-butyle :

Ce composé est préparé selon un mode opératoire analogue à celui décrit pour l'étape 4.1 de l'exemple 4, le bromure de 3-nitrophénacyle remplaçant le bromure de 4-azidophénacyle. Le composé attendu est obtenu sous forme d'une poudre de couleur crème avec un rendement de 44%.
RMN ¹H (δ ppm, DMSO): 4,96 (s, 2H); 7,69-7,75 (m, 4H); 7,90 (dd, 2H) ; 8,08 (dd, 2H).
MH+ = 319,2.

### 10.2) [4-(3-aminophényl)-1H-imidazol-2-yl]méthylcarbamate de tert-butyle :

Ce composé est préparé selon un mode opératoire analogue à celui décrit pour l'étape 4.2 de l'exemple 4, l'intermédiaire 10.1 remplaçant l'intermédiaire 4.1. Le composé attendu est obtenu sous forme d'une poudre de couleur crème avec un rendement de 89%.
RMN ¹H (δ ppm, DMSO): 1,39 (s, 9H); 4,2 (s, 2H); 6,43 (m, 1H) ; 6,84-7,01 (m, 3H) ; 7,26-7,34 (m, 2H).
MH+ = 289,2.

### 10.3) [4-(3-{[(1E)-amino(thién-2-yl)méthylène]amino}phényl)-1H-imidazol-2-yl]méthylcarbamate de tert-butyle:

Ce composé est préparé selon un mode opératoire analogue à celui décrit pour l'étape 4.3 de l'exemple 4, l'intermédiaire 10.2 remplaçant l'intermédiaire 4.2. Le composé attendu est obtenu sous forme d'une poudre blanche avec un rendement de 68%.
RMN ¹H (δ ppm, DMSO) : 1,39 (s, 9H) ; 4,15 (m, 2H) ; 6,34-6,62 (m, 3H) ; 7,08-7,74 (m, 8H) ; ;11,77 (s, 1H).

### 10.4) Chlorhydrate de N'-{3-[2-(aminométhyl)-1H-imidazol-4-yl]phényl}thiophène-2-carboximidamide :

Ce composé est préparé selon un mode opératoire analogue à celui décrit pour l'étape 4.4 de l'exemple 4, l'intermédiaire 10.3 remplaçant l'intermédiaire 4.3. Le composé attendu est obtenu sous forme d'une poudre blanche avec un rendement de 98%. Point de fusion : > 265 °C.
RMN ¹H (δ ppm, DMSO) : 4,40 (s, 2H) ; 7,38-7,45 (m, 2H) ; 7,64-7,68 (m, 1H) ; 7,95 (m, 2H) ; 8,12-8,23 (m, 3H) ; 9,06 (s large, 4H) ; 9,96 (s, 1H) ; 11,81 (s large, 1H).

### Exemple 11 : chlorhydrate de N'-{3-[2-({[(1E)-amino(thién-2-yl)méthylène]-amino}méthyl)-1H-imidazol-4-yl]phényl}thiophène-2-carboximidamide :

### 11.1) N'-{3-[2-({[(1E)-amino(thién-2-yl)méthylène)amino}méthyl)-1H-imidazol-4-yl]phényl}thiophène-2-carboximidamide :

Ce composé est préparé selon un mode opératoire analogue à celui décrit pour l'étape 4.3 de l'exemple 4, le composé de l'exemple 10 remplaçant l'intermédiaire 4.2. Le composé attendu est obtenu sous forme d'une poudre de couleur crème avec un rendement de 80%.
MH+ = 407,2.

### 11.2) Chlorhydrate de -N'-{3-[2-({[(1E)-amino(thién-2-yl)méthylène]amino}méthyl) 1H-imidazol-4 yl]phényl}thiophène-2-carboximidamide :

Ce composé est obtenu à partir de l'intermédiaire 11.1 selon un mode opératoire analogue à celui de l'étape 1.5 de l'exemple 1. Point de fusion : > 300 °C.
MH+ = 407,2.

*Le composé de l'exemple 12 est préparé selon un mode opératoire analogue à celui décrit pour le composé de l'exemple 11.*

### Exemple 12 : chlorhydrate de N'-{4-[2-({[(1E)-amino(thién-2-yl)méthylène]-amino}méthyl)-1H-imidazol-4-yl]phényl}thiophène-2-carboximidamide :

Point de fusion : 227-228 °C.
MH+ = 407,2.

### Exemple 13 : chlorhydrate de N-{3-[2-(2-cyclohexyléthyl)-1H-imidazol-4-yl]phényl}thiophène-2-carboximidamide :

### 13.1) 2-(2-cyclohexyléthyl)-4-(3-nitrophényl)-1H-imidazole :

Ce composé est préparé selon un mode opératoire analogue à celui décrit pour l'étape 4.1 de l'exemple 4, l'acide cyclohexyléthylcarboxylique et le bromure de 3-nitrophénacyle remplaçant respectivement la *N*-(*tert*-butoxycarbonyl)-glycine et le bromure de 4-azidophénacyle. Le composé attendu est obtenu sous forme d'une poudre jaune avec un rendement de 26%.
MH+ = 300,2.

### 13.2) 3-[2-(2-cyclohexyléthyl)-1H-imidazol-4-yl]aniline:

Ce composé est préparé selon un mode opératoire analogue à celui décrit pour l'étape 4.2 de l'exemple 4, l'intermédiaire 13.1 remplaçant l'intermédiaire 4.1. Le composé attendu est obtenu sous forme d'une poudre blanche avec un rendement de 93%.
MH+ = 270,2.

### 13.3) N-{3-[2-(2-cyclohexyléthyl)-1H-imidazol-4-yl]phényl}thiophène-2-carboximidamide:

Ce composé est préparé selon un mode opératoire analogue à celui décrit pour l'étape 4.3 de l'exemple 4, l'intermédiaire 13.2 remplaçant l'intermédiaire 4.2. Le composé attendu est obtenu sous forme d'une poudre blanche avec un rendement de 20%.
MH+ = 379,2.

### 13.4) Chlorhydrate de N-{3-[2-(2-cyclohexyléthyl)-1H-imidazol-4-yl]phényl}-thiophène-2-carboximidamide :

Ce composé est obtenu à partir de l'intermédiaire 13.3 selon un mode opératoire analogue à celui de l'étape 1.5 de l'exemple 1. Point de fusion : > 191-193 °C.
MH+ = 379,2.

*Les composés des exemples 14 à 19 sont préparés selon un mode opératoire analogue à celui décrit pour le composé de l'exemple 13.*

### Exemple 14 : chlorhydrate de N'-{3-[2-(1-pentylhexyl)-1H-imidazol-4-yl]phényl}thiophène-2-carboximidamide :

Point de fusion : 163,3 °C.
MH+ = 423,2.

### Exemple 15 : chlorhydrate de N-{4-[2-(2-cyclohexyléthyl)-1H-imidazol-4-yl]phényl}thiophène-2-carboximidamide :

Point de fusion : 196,2 °C.
MH+ = 379,2.

### Exemple 16 : chlorhydrate de N'-{3-[2-(cyclohexylméthyl)-1H-imidazol-4-yl]phényl}thiophène-2-carboximidamide :

Mousse jaune pâle.
MH+ = 365,2 ; t.r. = 7,40 min.

### Exemple 17 : chlorhydrate de N'-{3-[2-(3-cyclohexylpropyl)-1H-imidazol-4-yl]phényl}thiophène-2-carboximidamide :

Point de fusion : 180-181 °C.
MH+ = 393,2.

### Exemple 18 : chlorhydrate de N-[3-(2-hexyl-1H-imidazol-4-yl)phényl]thiophène-2-carboximidamide :

Mousse jaune pâle.
MH+ = 353,2 ; t.r. = 7,40 min.

### Exemple 19 : chlorhydrate de N-{4-[2-(2-cyclohexyléthyl)-1H-imidazol-4-yl]phényl}-N'-nitroguanidine :

Point de fusion : 185-186 °C.
MH+ = 357,2.

*Les composés des exemples 20 à 23 sont préparés selon un mode opératoire analogue à celui décrit pour le composé de l'exemple 4.*

### Exemple 20 : chlorhydrate de N'-(4-{2-[(cycloheptylamino)méthyl]-1H-imidazol-4-yl}phényl)thiophène-2-carboximidamide :

Point de fusion : 264-265 °C.

### Exemple 21: chlorhydrate de N'-(4-{2-[(méthylamino)méthyl]-1H-imidazol-4-yl}phényl)thiophène-2-carboximidamide :

Point de fusion : > 250 °C.

### Exemple 22: chlorhydrate de N'-(4-{2-[(cyclobutylamino)méthyl]-1H-imidazol-4-yl}phényl)thiophène-2-carboximidamide :

Point de fusion : 263-264 °C.

### Exemple 23: chlorhydrate de N-[4-(2-([(2,2-diphényléthyl)amino]méthyl)-1H-imidazol-4-yl)phényl]thiophène-2-carboximidamide :

Point de fusion : > 250 °C [le réactif utilisé lors de la dernière étape, le 3,3-diphénylpropanal, est préparé à partir des composés commerciaux adaptés selon un protocole similaire à celui décrit dans J. Org. Chem. (1990), 55(17), 5078-88].

*Le composé de l'exemple 24 est préparé selon un mode opératoire analogue à celui décrit pour le composé de l'exemple 11.*

### Exemple 24: chlorhydrate de N'-{3-[2-(2-{[(1E)-amino(thién-2-yl)méthylène]amino}éthyl)-1H-imidazol-4-yl]phényl}thiophène-2-carboximidamide :

Point de fusion : > 245 °C.

*Les composés des exemples 25 à 2 7 sont préparés selon un mode opératoire analogue à celui décrit pour le composé de l'exemple 13.*

### Exemple 25: chlorhydrate de N'-(3-{2-[(phénylthio)méthyl]-1H-imidazol-4-yl}phényl)thiophène-2-carboximidamide :

Ce composé est obtenu sous forme d'une mousse jaune pâle.
MH+ = 391,1; t.r. = 7,30 min.

### Exemple 26: chlorhydrate de N'-(4-{2-[(phénylthio)méthyl]-1H-imidazol-4-yl}phényl)thiophène-2-carboximidamide :

Ce composé est obtenu sous forme d'une mousse jaune pâle.
MH+ = 391,1; t.r. = 7,30 min.

### Exemple 27: chlorhydrate de N'-{3-[2-(4-isobutylbenzyl)-1H-imidazol-4-yl]phényl}thiophène-2-carboximidamide :

Point de fusion : 214-216 °C [la préparation du composé de départ, l'acide (4-isobutylphényl)acétique, a été décrit dans la demande PCT WO 02/102375 - voir exemple 1, étape 1 de ce document].

### Étude pharmacologique des produits de l'invention

### Test de liaison sur les canaux sodiques de cortex cérébraux de rat

Le test consiste à mesurer l'interaction des composés vis-à-vis de la liaison de la batrachotoxine tritiée sur les canaux sodiques dépendants du voltage selon le protocole décrit par Brown (J. Neurosci. (1986), 6, 2064-2070).

### Préparation des homogénats de cortex cérébraux de rat

Les cortex cérébraux de rat Sprague-Dawley de 230-250 g (Charles River, France) sont prélevés, pesés et homogénéisés à l'aide d'un broyeur Potter muni d'un piston en téflon (10 allers/retours) dans 10 volumes de tampon d'isolement dont la composition est la suivante (sucrose 0,32 M ; K₂HPO₄ 5 mM ; pH 7,4). L'homogénat subit une première centrifugation à 1000 g pendant 10 minutes. Le surnageant est prélevé et centrifugé à 20000 g pendant 15 minutes. Le culot est repris dans le tampon d'isolement et centrifugé à 20000 g pendant 15 minutes. Le culot obtenu est remis en suspension dans du tampon d'incubation (HEPES 50 mM ; KCl 5,4 mM ; MgSO₄ 0,8 mM ; glucose 5,5 mM ; chlorure de choline 130 mM pH 7,4) puis aliquoté et conservé à -80°C jusqu'au jour du dosage. La concentration finale en protéines est comprise entre 4 et 8 mg/ml. Le dosage de protéines se fait par un kit commercialisé par BioRad (France).

### Mesure de la liaison de la batrachotoxine tritiée

La réaction de liaison se fait en incubant pendant 1 h 30 à 25 °C 100 µl d'homogénat de cortex de rat contenant 75 µg de protéines avec 100 µl de [³H] batrachotoxine-A 20-alpha benzoate (37,5 Ci/mmol, NEN) à 5 nM (concentration finale), 200 µl de tétrodotoxine à 1 µM (concentration finale) et de venin de scorpion à 40 µg/ml (concentration finale) et 100 µl de tampon d'incubation seul ou en présence des produits à tester aux différentes concentrations. La liaison non spécifique est déterminée en présence de 300 µM de vératridine et la valeur de cette liaison non spécifique est soustraite à toutes les autres valeurs. Les échantillons sont ensuite filtrés à l'aide d'un Brandel (Gaithersburg, Maryland, USA) en utilisant des plaques Unifilter GF/C préincubées avec 0,1 % de polyéthylène imine (20 µl/puits) et rincées 2 fois avec 2 ml de tampon de filtration (HEPES 5 mM ; CaCl₂ 1,8 mM ; MgSO₄ 0,8 mM ; chlorure de choline 130 mM ; pH 7,4). Après avoir ajouté 20 µl de Microscint 0 ^{®}, la radioactivité est comptée à l'aide d'un compteur à scintillation liquide (Topcount, Packard). La mesure est réalisée en duplicat. Les résultats sont exprimés en % de la liaison spécifique de la batrachotoxine tritiée par rapport au témoin.

### Résultats

Les composés des exemples 1 à 9, 11 à 20 et 22 à 27 décrits ci-dessus présentent tous une CI₅₀ inférieure ou égale à 10 µM.

### Étude des effets sur la NO synthase constitutive neuronale de cervelet de rat

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (Proc. Natl. Acad. Sci. USA, (1990) 87: 682-685).

### Préparation des homogénats de cervelets de rat

Des cervelets de rats Sprague-Dawley (300 g - Charles River) sont rapidement prélevés, pesés et homogénéisés dans 5 volumes de tampon d'extraction (HEPES 50 mM, EDTA 1 mM, pH 7,4, pepstatine A 10 mg/ml, leupeptine 10 mg/ml). Les homogénats sont ensuite centrifugés à 35000 g pendant 1 heure à 4 °C. Les surnageants sont ensuite passés sur une colonne de résine DOWEX 50W-X8, forme sodium reprise dans le tampon d'extraction, afin d'éliminer l'arginine endogène. La préparation obtenue est aliquotée et conservée à -80 °C.

### Dosage de l'activité NOS neuronale

Le tampon d'incubation est composé de 100 mM d'HEPES (pH 7,4), 2 mM d'EDTA, 2,5 mM de CaCl₂, 2 mM de dithiotréitol, 2 mM de NADPH réduit, 10 µg/ml de calmoduline, 10 µM de FAD, 10 µM de FMN, et 10 µM de BH4. Les produits à tester sont dilués dans ce tampon. La réaction est réalisée en incubant 15 minutes à 37°C 100 µl de tampon d'incubation contenant ou non les inhibiteurs, 25 µl d'une solution contenant 62,5 nM de [³H]L-arginine (Activité spécifique: 56,4 Ci/mmol, Perkin-Elmer) et 25 µM de L-arginine non radioactive, 25 µl de tampon d'incubation, et 50 µl de préparation enzymatique diluée 10 fois dans du tampon HEPES 50 mM. La réaction est arrêtée avec 2 ml de tampon HEPES 20 mM pH 5,5 contenant 2 mM d'EDTA. La totalité des échantillons est passée sur une colonne de 1 ml de résine DOWEX 50W -X8, forme sodium, reprise dans le tampon d'arrêt. Après avoir ajouté 16 ml de liquide scintillant (Ultima Gold, Packard), la radioactivité est quantifiée par un compteur à scintillation liquide (Winspectral 1410, Wallac). Les mesures sont réalisées en duplicats. Chaque série de mesure comprend 2 tubes ne contenant pas d'enzyme (blanc réactionnel dont la valeur est soustraite de chaque mesure) et 2 tubes ne contenant pas d'inhibiteurs (témoin réactionnel). Les résultats sont exprimés en pourcentage d'inhibition de la réaction enzymatique (valeur du témoin réactionnel).

### Résultats

Les composés des exemples 1 à 13, 15, 16 et 18 à 26 décrits ci-dessus présentent tous une CI₅₀ inférieure ou égale à 10 µM.

## Revendications

1. Composé de formule générale (i) 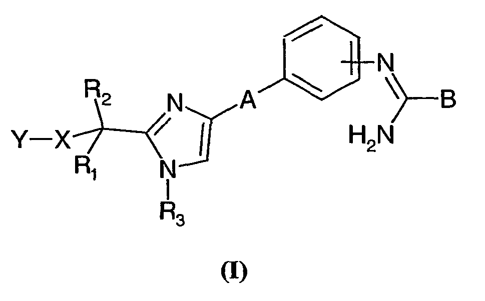 sous forme de mélange racémique, d'énantiomère ou de toute combinaison de ces formes,
dans laquelle
R₁ représente un atome d'hydrogène ou un radical alkyle, cycloalkyle, cycloalkylalkyle, ou encore l'un des radicaux aryle ou aralkyle dont le noyau aromatique est éventuellement substitué de 1 à 3 fois par des substituants choisis indépendamment parmi un atome halogène et un radical alkyle ou alkoxy ;
R₂ représente un atome d'hydrogène ou un radical alkyle ;
R₃ représente un atome d'hydrogène ou un radical alkyle ou aralkyle ;
X représente une liaison ou un radical alkylène linéaire ou ramifié comptant de 1 à 5 atomes de carbone ;
Y représente un atome d'hydrogène, un radical cycloalkyle, un radical NR₄R₅, OR₁₄ ou SR₁₅ ou un radical 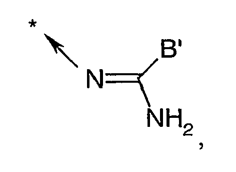 ou encore Y représente un radical aryle éventuellement substitué de 1 à 3 fois par des substituants choisis indépendamment parmi un atome halogène et un radical alkyle ou alkoxy ;
A représente une liaison ou le radical phénylène ;
B et B' sont choisis indépendamment parmi un radical alkyle, un radical cycloalkyle, un radical NR₆R₇ ou SR₈, un radical aryle carbocyclique ou un radical aryle hétérocyclique à 5 ou 6 chaînons contenant de 1 à 4 hétéroatomes choisis parmi O, S et N, lesdits radicaux aryle carbocyclique et hétérocyclique étant éventuellement substitués par un à trois groupes choisis indépendamment parmi les radicaux alkyle, alkényle ou alkoxy,
R₄ représente un atome d'hydrogène ou un radical alkyle, cycloalkyle, cycloalkylalkyle, -C(O)R₉, -C(O)OR₉, -C(O)NHR₉ ou -SO₂R₉ ou encore l'un des radicaux aryle ou aralkyle dont le noyau aromatique est éventuellement substitué de 1 à 3 fois par des substituants choisis indépendamment parmi un atome halogène et un radical alkyle ou alkoxy, ou R₄ représente un radical *bis*-phénylalkyle,
R₅ représente un atome d'hydrogène ou un radical alkyle, aryle ou aralkyle, ou encore R₄ et R₅ forment avec l'atome d'azote qui les porte un hétérocycle non aromatique de cinq à sept chaînons comptant de 1 à 2 hétéroatomes, les éléments pour compléter l'hétérocycle étant choisis indépendamment parmi un groupe composé de -CHR₁₀-, -NR₁₁-, -O- et -S- ;
R₆ et R₇ représentent indépendamment un atome d'hydrogène ou un radical alkyle, alkényle ou alkynyle,
ou bien R₆ représente un radical nitro et R₇ représente un atome d'hydrogène,
ou encore R₆ et R₇ forment avec l'atome d'azote qui les porte un hétérocycle non aromatique de cinq à six chaînons, les éléments pour compléter l'hétérocycle étant choisis indépendamment parmi un groupe composé de -CH₂-, -NR₁₂-, -O- et -S- ;
R₈ représente un radical alkyle linéaire ou ramifié ayant de 1 à 6 atomes de carbone éventuellement substitué de 1 à 3 fois par un ou des substituants choisis indépendamment parmi un atome halogène et les radicaux -OH, amino, cyano et aryle ;
R₉ représente un radical alkyle, haloalkyle, cycloalkyle ou cycloalkylalkyle, ou encore l'un des radicaux aralkyle ou aryle carbocyclique ou hétérocyclique dont le noyau aromatique est éventuellement substitué de 1 à 3 fois par des substituants choisis indépendamment parmi un atome halogène et un radical alkyle ou alkoxy ;
R₁₀ représente un atome d'hydrogène ou un radical alkyle ou aryle éventuellement substitué de 1 à 3 fois par des substituants choisis indépendamment parmi un atome halogène et un radical alkyle ou alkoxy,
R₁₁ représente un atome d'hydrogène, un radical alkyle, un radical cycloalkyle, un radical cycloalkylalkyle, un radical -C(O)R₁₃, un radical -C(O)OR₁₃, un radical-SO₂R₁₃, un radical -C(O)NHR₁₃, ou encore l'un des radicaux aryle ou aralkyle dont le noyau aromatique est éventuellement substitué de 1 à 3 fois par des substituants choisis indépendamment parmi un atome halogène et un radical alkyle ou alkoxy ;
R₁₂ représente un atome d'hydrogène ou un radical alkyle ;
R₁₃ représente un radical alkyle, un radical haloalkyle ou encore l'un des radicaux aralkyle ou aryle carbocyclique ou hétérocyclique dont le noyau aromatique est éventuellement substitué de 1 à 3 fois par des substituants choisis indépendamment parmi un atome halogène et un radical alkyle ou alkoxy ;
R₁₄ représente un radical alkyle, le radical phényle ou un radical aralkyle ; et enfin
R₁ représente un radical alkyle, le radical phényle ou un radical aralkyle;
étant entendu :
- qu'un radical alkyle ou alkoxy, lorsqu'il n'est pas donné plus de précision, est linéaire ou ramifié et compte de 1 à 12 atomes de carbone ;
- qu'un radical alkényle ou alkynyle, lorsqu'il n'est pas donné plus de précision, est linéaire ou ramifié et compte de 2 à 6 atomes de carbone ;
- qu'un radical cycloalkyle, lorsqu'il n'est pas donné plus de précision, compte de 3 à 7 atomes de carbone ;
ou sel d'un composé de formule générale **(I).**

2. Composé de formule générale **(I)** selon la revendication 1, **caractérisé en ce que** X représente une liaison ou un radical alkylène linéaire ou ramifié comptant de 1 à 5 atomes de carbone et Y représente un radical NR₄R₅ ;
ou sel de ce composé.

3. Composé de formule générale **(I)** selon la revendication 1, **caractérisé en ce que** X représente une liaison ou un radical alkylène linéaire ou ramifié comptant de 1 à 5 atomes de carbone et Y représente un radical 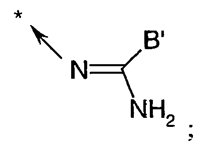 ou sel de ce composé.

4. Composé de formule générale **(I)** selon la revendication 1, **caractérisé en ce que** X représente une liaison ou un radical alkylène linéaire ou ramifié comptant de 1 à 5 atomes de carbone et Y représente un radical cycloalkyle ou un radical aryle éventuellement substitué de 1 à 3 fois par des substituants choisis indépendamment parmi un atome halogène et un radical alkyle ou alkoxy ;
ou sel de ce composé.

5. Composé de formule générale **(I)** selon la revendication 1, **caractérisé en ce que** X représente une liaison et Y représente un atome d'hydrogène tandis que l'un au moins de R₁ et R₂ représente un radical choisi parmi les radicaux alkyle, cycloalkyle ou cycloalkylalkyle ;
ou sel de ce composé.

6. Composé de formule générale **(I)** selon la revendication 1, **caractérisé en ce qu'**il est choisi parmi les composés suivants :
- 2-[4-(4-{[(1Z)-amino(thién-2-yl)méthylène]-amino}phényl)-1*H*-imidazol-2-yl]éthylcarbamate de butyle ;
- 2-[4-(3-{[(1E)-amino(thién-2-yl)méthylène]-amino}phényl)-1H-imidazol-2-yl]éthylcarbamate de butyle ;
- 2-[4-(4'-{[(1Z)-amino(thién-2-yl)méthylène]amino}-1,1'-biphényl-4-yl)-1H-imidazol-2-yl]éthylcarbamate de butyle ;
- N'-(4-{2-[(cyclohexylamino)méthyl]-1H-imidazol-4-yl}phényl)thiophène-2-carboximidamide ;
- *N*'-(4-{2-[2-(cyclohexylamino)éthyl]-1*H*-imidazol-4-yl}phényl)thiophène-2-carboximidamide ;
- *N*'-(3-(2-[(cyclohexylamino)méthyl]-1*H*-imidazol-4-yl}phényl)thiophène-2-carboximidamide ;
- *N*'-[4-(2-{[cyclohexyl(méthyl)amino]méthyl}-1*H*-imidazol-4-yl)phényl]thiophène-2-carboximidamide ;
- *N*'-(4-{2-[(dibenzylamino)méthyl]-1*H* imidazol-4-yl}phényl)thiophène-2-carboximidamide ;
- *N*'-(4-{2-[(benzylamino)méthyl]-1*H*-imidazol-4-yl}phényl)thiophène-2-carboximidamide ;
- *N*'-{3-[2-(aminométhyl)-1H-imidazol-4-yl]phényl}thiophène-2-carboximidamide ;
- *N*'-{3-[2-({[(1*E*)-amino(thién-2-yl)méthylène]-amino}méthyl)-1*H*-imidazol-4-yl]phényl}thiophène-2-carboximidamide ;
- *N*'-{4-[2-({[(1*E*)-amino(thién-2-yl)méthylène]-amino}méthyl)-1*H*-imidazol-4-yl]phényl}thiophène-2-carboximidamide ;
- *N*-{3-[2-(2-cyclohexyléthyl)-1*H*-imidazol-4-yl]phényl}thiophène-2-carboximidamide ;
- *N*'-{3-[2-(1-pentylhexyl)-1*H*-imidazol-4-yl]phényl}thiophène-2-carboximidamide;
- *N*'-{4-[2-(2-cyclohexyléthyl)-1*H*-imidazol-4-yl]phényl}thiophène-2-carboximidamide ;
- *N*'-{3-[2-(cyclohexylméthyl)-1*H*-imidazol-4-yl]phényl}thiophène-2-carboximidamide ;
- *N'*-{3-[2-(3-cyclohexylpropyl)-177-imidazol-4-yl]phényl}tbiophène-2-carboximidamide ;
- *N*'-[3-(2-hexyl-1*H*-imidazol-4-yl)phényl]thiophène-2-carboximidamide ;
- *N-*{4-[2-(2-cyclohexyléthyl)-1*H*-imidazol-4-yl]phényl}-*N*''-nitroguanidine ;
- *N*'-(4-{2-[(cycloheptylamino)méthyl]-1*H*-imidazol-4-yl}phényl)thiophène-2-carboximidamide;
- *N*'-(4-{2-[(méthylamino)méthyl]-1*H*-imidazol-4-yl}phényl)thiophène-2-carboximidamide;
- *N*'-(4-{2-[(cyclobutylamino)méthyl]-1H-imidazol-4-yl}phényl)thiophène-2-carboximidamide;
- *N'*-[4-(2-{[(2,2-diphényléthyl)amino]méthyl}-1H-imidazol-4-yl)phényl]thiophène-2-carboximidamide;
- *N*'-{3-[2-(2-{[(1E)-amino(thién-2-yl)méthylène]amino}éthyl)-1H-imidazol-4-yl]phényl}thiophène-2-carboximidamide;
- *N*'-(3-{2-[(phénylthio)méthyl]-1H-imidazol-4-yl}phényl)thiophène-2-carboximidamide;
- *N*'-(4-{2-[(phénylthio)méthyl]-1H-imidazol-4-yl}phényl)thiophène-2-carboximidamide;
- *N*-{3-[2-(4-isobutylbenzyl)-1*H*-imidazol-4-yl]phényl}thiophène-2-carboximidamide;
ou sel dudit composé de formule générale **(I).**

7. A titre de médicament, un composé de formule générale **(I)** telle que définie dans la revendication 1, ou un sel pharmaceutiquement acceptable dudit composé.

8. Composition pharmaceutique contenant, à titre de principe actif, un composé de formule générale **(I)** telle que définie dans la revendication 1, ou un sel pharmaceutiquement acceptable dudit composé.

9. Utilisation d'un composé de formule générale **(I)** telle que définie dans la revendication 1, ou un sel pharmaceutiquement acceptable dudit composé, pour préparer un médicament destiné à traiter ou prévenir un désordre choisi / une maladie choisie parmi les désordres suivants / maladies suivantes : les douleurs, la sclérose en plaques, les troubles du système nerveux central ou périphérique, les troubles cardiovasculaires, les troubles du muscle squelettique et des jonctions neuromusculaires, les maladies inflammatoires, les pertes d'audition d'origine traumatique, acoustique ou toxique et les acouphènes, les complications liées aux maladies auto-immunes et virales et les maladies neurologiques associées à des intoxications, à des traitements ou à des désordres d'origine génétique.

10. Utilisation selon la revendication 9, **caractérisée en ce que** le médicament préparé est destiné à traiter ou prévenir les douleurs.

## Claims

1. Compound of general formula (i) 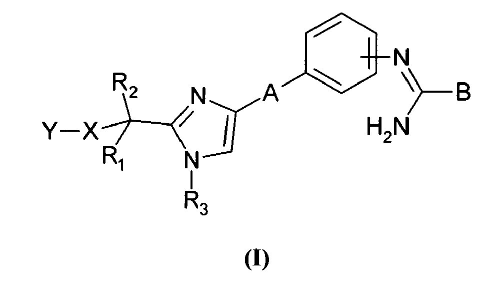 in the form of racemic, enantiomeric mixture or any combination of these forms,
in which
R₁ represents a hydrogen atom or an alkyl, cycloalkyl, cycloalkylalkyl radical, or also one of the aryl or aralkyl radicals, the aromatic ring of which is optionally substituted from once to 3 times by substituents chosen independently from a halogen atom and an alkyl or alkoxy radical;
R₂ represents a hydrogen atom or an alkyl radical;
R₃ represents a hydrogen atom or an alkyl or aralkyl radical;
X represents a bond or a linear or branched alkylene radical containing from 1 to 5 carbon atoms;
Y represents a hydrogen atom, a cycloalkyl radical, an NR₄R₅, OR₁₄ or SR₁₅ radical or a 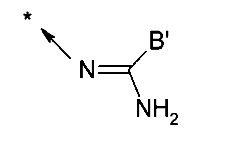 radical, or also Y represents an aryl radical optionally substituted from once to 3 times by substituents chosen independently from a halogen atom and an alkyl or alkoxy radical;
A represents a bond or the phenylene radical;
B and B' are chosen independently from an alkyl radical, a cycloalkyl radical, an NR₆R₇ or SR₈ radical, a carbocyclic aryl radical or a heterocyclic aryl radical with 5 or 6 members containing from 1 to 4 heteroatoms chosen from O, S and N, said carbocyclic and heterocyclic aryl radicals being optionally substituted by one to three groups chosen independently from the alkyl, alkenyl or alkoxy radicals,
R₄ represents a hydrogen atom or an alkyl, cycloalkyl, cycloalkylalkyl, -C(O)R₉, -C(O)OR₉, -C(O)NHR₉ or -SO₂R₉ radical, or also one of the aryl or aralkyl radicals the aromatic ring of which is optionally substituted from once to 3 times by substituents chosen independently from a halogen atom and an alkyl or alkoxy radical, or R₄ represents a bis-phenylalkyl radical,
R₅ represents a hydrogen atom or an alkyl, aryl or aralkyl radical,
or also R₄ and R₅ form with the nitrogen atom which carries them a non-aromatic heterocycle with five to seven members containing from 1 to 2 heteroatoms, the elements for completing the heterocycle being chosen independently from a group comprising
-CHR₁₀-, -NR₁₁-, -O- and -S-;
R₆ and R₇ represent independently a hydrogen atom or an alkyl, alkenyl or alkynyl radical,
or R₆ represents a nitro radical and R₇ represents a hydrogen atom,
or also R₆ and R₇ form with the nitrogen atom which carries them a non-aromatic heterocycle with five to six members, the elements for completing the heterocycle being chosen independently from a group comprising -CH₂-, -NR₁₂-, -O- and -S-;
R₈ represents a linear or branched alkyl radical having 1 to 6 carbon atoms optionally substituted from once to 3 times by one or more substituents chosen independently from a halogen atom and the -OH, amino, cyano and aryl radicals;
R₉ represents an alkyl, haloalkyl, cycloalkyl or cycloalkylalkyl radical, or also one of the carbocyclic or heterocyclic aralkyl or aryl radicals the aromatic ring of which is optionally substituted from once to 3 times by substituents chosen independently from a halogen atom and an alkyl or alkoxy radical;
R₁₀ represents a hydrogen atom or an alkyl or aryl radical optionally substituted from once to 3 times by substituents chosen independently from a halogen atom and an alkyl or alkoxy radical,
R₁₁ represents a hydrogen atom, an alkyl radical, a cycloalkyl radical, a cycloalkylalkyl radical, a -C(O)R₁₃ radical, a -C(O)OR₁₃ radical, an -SO₂R₁₃ radical, a -C(O)NHR₁₃ radical, or also one of the aryl or aralkyl radicals, the aromatic ring of which is optionally substituted from once to 3 times by substituents chosen independently from a halogen atom and an alkyl or alkoxy radical;
R₁₂ represents a hydrogen atom or an alkyl radical;
R₁₃ represents an alkyl radical, a haloalkyl radical or also one of the carbocyclic or heterocyclic aralkyl or aryl radicals the aromatic ring of which is optionally substituted from once to 3 times by substituents chosen independently from a halogen atom and an alkyl or alkoxy radical;
R₁₄ represents an alkyl radical, the phenyl radical or an aralkyl radical; and finally
R₁₅ represents an alkyl radical, the phenyl radical or an aralkyl radical;
it being understood:
- that an alkyl or alkoxy radical, unless otherwise specified, is linear or branched and contains from 1 to 12 carbon atoms;
- that an alkenyl or alkynyl radical, unless otherwise specified, is linear or branched and contains from 2 to 6 carbon atoms;
- that a cycloalkyl radical, unless otherwise specified, comprises from 3 to 7 carbon atoms;
or a salt of a compound of general formula **(I).**

2. Compound of general formula **(I)** according to claim 1, **characterized in that** X represents a bond or a linear or branched alkylene radical containing from 1 to 5 carbon atoms and Y represents an NR₄R₅ radical;
or a salt of this compound.

3. Compound of general formula **(I)** according to claim 1, **characterized in that** X represents a bond or a linear or branched alkylene radical containing from 1 to 5 carbon atoms and Y represents a 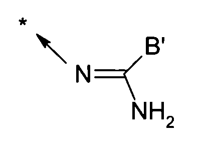 radical;
or a salt of this compound.

4. Compound of general formula **(I)** according to claim 1, **characterized in that** X represents a bond or a linear or branched alkylene radical containing from 1 to 5 carbon atoms and Y represents a cycloalkyl radical or an aryl radical optionally substituted from once to 3 times by substituents chosen independently from a halogen atom and an alkyl or alkoxy radical;
or a salt of this compound.

5. Compound of general formula **(I)** according to claim 1, **characterized in that** X represents a bond and Y represents a hydrogen atom whilst at least one of R₁ and R₂ represents a radical chosen from the alkyl, cycloalkyl or cycloalkylalkyl radicals;
or a salt of this compound.

6. Compound of general formula **(I)** according to claim 1, **characterized in that** it is chosen from the following compounds:
- butyl-2-[4-(4-{[(1Z)-amino(thien-2-yl)methylene]-amino}phenyl)-1*H*-imidazol-2-yl]ethylcarbamate;
- butyl-2-[4-(3-{[(1E)-amino(thien-2-yl)methylene]-amino}phenyl)-1H-imidazol-2-yl]ethylcarbamate;
- butyl-2-[4-(4'-{[(1Z)-amino(thien-2-yl)methylene]amino}-1,1'-biphenyl-4-yl)-1H-imidazol-2-yl]ethylcarbamate;
- N'-(4-{2-[(cyclohexylamino)methyl]-1H-imidazol-4-yl}phenyl)thiophene-2-carboximidamide ;
- *N*'-(4-{2-[2-(cyclohexylamino)ethyl]-1*H*-imidazol-4-yl}phenyl)thiophene-2-carboximidamide ;
- *N*'-(3-{2-[(cyclohexylamino)methyl]-1*H*-imidazol-4-yl}phenyl)thiophene-2-carboximidamide;
- *N*'-[4-(2-{[cyclohexyl(methyl)amino]methyl}-1*H*-imidazol-4-yl)phenyl]thiophene-2-carboximidamide;
- *N*'-(4-{2-[(dibenzylamino)methyl]-1*H*-imidazol-4-yl}phenyl)thiophene-2-carboximidamide;
- *N*'-(4-{2-[(benzylamino)methyl]-1*H*-imidazol-4-yl}phenyl)thiophene-2-carboximidamide;
- *N'*-{3-[2-(aminomethyl)-1H-imidazol-4-yl]phenyl}thiophene-2-carboximidamide;
- *N*'-{3-[2-({[(1*E*)-amino(thien-2-yl)methylene]-amino}methyl)-1*H*-imidazol-4-yl]phenyl}thiophene-2-carboximidamide;
- *N'-*{4-[2-({[(1*E*)-amino(thien-2-yl)methylene]-amino}methyl)-1*H*-imidazol-4-yl]phenyl}thiophene-2-carboximidamide;
- *N*-{3-[2-(2-cyclohexylethyl)-1*H*-imidazol-4-yl]phenyl}thiophene-2-carboximidamide;
- *N*'-{3-[2-(1-pentylhexyl)-1*H*-imidazol-4-yl]phenyl}thiophene-2-carboximidamide;
- *N*'-{4-[2-(2-cyclohexylethyl)-1*H*-imidazol-4-yl]phenyl}thiophene-2-carboximidamide;
- *N*'-{3-[2-(cyclohexylmethyl)-1*H*-imidazol-4-yl]phenyl}thiophene-2-carboximidamide;
- *N*'-{3-[2-(3-cyclohexylpropyl)-1*H*-imidazol-4-yl]phenyl}thiophene-2-carboximidamide;
- *N*'-[3-(2-hexyl-1*H*-imidazol-4-yl)phenyl]thiophene-2-carboximidamide;
- *N-*{4-[2-(2-cyclohexylethyl)-1*H*-imidazol-4-yl]phenyl}-*N*''-nitroguanidine;
- *N*'-(4-{2-[(cycloheptylamino)methyl]-1*H*-imidazol-4-yl}phenyl)thiophene-2-carboximidamide;
- *N*'-(4-{2-[(methylamino)methyl]-1*H*-imidazol-4-yl}phenyl)thiophene-2-carboximidamide;
- *N*'-(4-{2-[(cyclobutylamino)methyl]-1*H*-imidazol-4-yl}phenyl)thiophene-2-carboximidamide;
- *N*'-[4-(2-{[(2,2-diphenylethyl)amino]methyl}-1*H* imidazol-4-yl)phenyl]thiophene-2-carboximidamide;
- *N*'-{3-[2-(2-{[(1E)-amino(thien-2-yl)methylene]amino}ethyl)-1*H*-imidazol-4-yl]phenyl}thiophene-2-carboximidamide;
- *N*'-(3-{2-[(phenylthio)methyl]-1H- imidazol-4-yl}phenyl)thiophene-2-carboximidamide;
- *N*'-(4-{2-[(phenylthio)methyl]-1H-imidazol-4-yl}phenyl)thiophene-2-carboximidamide;
- *N*'-{3-[2-(4-isobutylbenzyl)-1*H*-imidazol-4-yl]phenyl}thiophene-2-carboximidamide;
or a salt of said compound of general formula **(I).**

7. As a medicament, a compound of general formula **(I)** as defined in claim 1, or a pharmaceutically acceptable salt of said compound.

8. Pharmaceutical composition containing, as active ingredient, a compound of general formula **(I)** as defined in claim 1, or a pharmaceutically acceptable salt of said compound.

9. Use of a compound of general formula **(I)** as defined in claim 1, or a pharmaceutically acceptable salt of said compound, for preparing a medicament intended to treat or prevent a disorder/disease chosen from the following disorders/diseases: pain, multiple sclerosis, disorders of the central or peripheral nervous system, cardiovascular disorders, disorders of the skeletal muscle and of the neuromuscular joints, inflammatory diseases, hearing losses of traumatic, acoustic or toxic origin and tinnitus, complications linked with auto-immune and viral diseases and the neurological diseases associated with intoxication, treatments or disorders of genetic origin.

10. Use according to claim 9, **characterized in that** the medicament prepared is intended to treat or prevent pain.

## Patentansprüche

1. Verbindung der allgemeinen Formel (i) 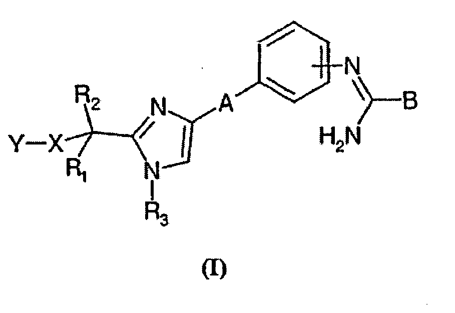 in Form einer racemischen Mischung, in Enantiomerenform oder in jeder Kombination dieser Formen,
in der
R₁ ein Wasserstoffatom oder einen Rest Alkyl, Cycloalkyl, Cycloalkylalkyl oder einen der Reste Aryl oder Aralkyl darstellt, deren aromatischer Ring gegebenenfalls 1 bis 3 mal mit Substituenten substituiert ist, die unabhängig voneinander aus einem Halogenatom und einem Rest Alkyl oder Alkoxy ausgewählt sind;
R₂ ein Wasserstoffatom oder einen Alkylrest darstellt;
R₃ ein Wasserstoffatom oder einen Alkyl- oder Aralkylrest darstellt;
X eine Bindung oder einen linearen oder verzweigten Alkylenrest mit 1 bis 5 Kohlenstoffatomen darstellt;
Y ein Wasserstoffatom, einen Cycloalkylrest, einen Rest NR₄R₅, OR₁₄ oder SR₁₅ oder einen Rest 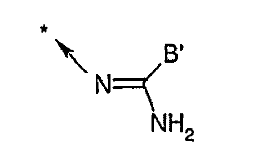 darstellt oder Y einen Arylrest darstellt, der gegebenenfalls 1 bis 3 mal mit Substituenten substituiert ist, die unabhängig voneinander aus einem Halogenatom und einem Alkyl- oder Alkoxyrest ausgewählt sind;
A eine Bindung oder den Phenylenrest darstellt;
B und B' unabhängig voneinander ausgewählt sind aus einem Alkylrest, einem Cycloalkylrest, einem Rest NR₆R₇ oder SR₈, einem carbocyclischen Arylrest oder einem heterocyclischen Arylrest mit 5 oder 6 Gliedern, enthaltend 1 bis 4 Heteroatome, die aus O, S und N ausgewählt sind, wobei die carbocyclischen und heterocyclischen Arylreste gegebenenfalls mit einer bis drei Gruppen substituiert sind, die unabhängig voneinander aus den Resten Alkyl, Alkenyl oder Alkoxy ausgewählt sind,
R₄ ein Wasserstoffatom oder einen Rest Alkyl, Cycloalkyl, Cycloalkylalkyl, -C(O)R₉, -(CO)OR₉, -C(O)NHR₉ oder -SO₂R₉ oder einen der Reste Aryl oder Aralkyl darstellt, deren aromatischer Ring gegebenenfalls 1 bis 3 mal mit Substituenten substituiert ist, die unabhängig voneinander aus einem Halogenatom und einem Alkyl- oder Alkoxyrest ausgewählt sind, oder R₄ einen Rest bis-Phenylalkyl darstellt,
R₅ ein Wasserstoffatom oder einen Rest Alkyl, Aryl oder Aralkyl darstellt,
oder R₄ und R₅ mit dem sie tragenden Stickstoffatom einen nicht-aromatischen Heterocyclus von fünf bis sieben Gliedern mit 1 bis 2 Heteroatomen bilden, wobei die Elemente zur Vervollständigung des Heterocyclus unabhängig voneinander aus einer Gruppe ausgewählt sind, die aus -CHR₁₀-, -NR₁₁-, -O- und -S- besteht;
R₆ und R₇ unabhängig voneinander ein Wasserstoffatom oder einen Rest Alkyl, Alkenyl oder Alkinyl darstellen,
oder R₆ einen Nitrorest und R₇ ein Wasserstoffatom darstellt,
oder R₆ und R₇ mit dem sie tragenden Stickstoffatom einen nicht-aromatischen Heterocyclus von fünf bis sechs Gliedern bilden, wobei die Elemente zur Vervollständigung des Heterocyclus unabhängig voneinander aus einer Gruppe ausgewählt sind, die aus -CH₂-, -NR₁₂-, -O- und -S- besteht;
R₈ einen linearen oder verzweigten Alkylrest mit 1 bis 6 Kohlenstoffatomen darstellt, der gegebenenfalls 1 bis 3 mal mit einem oder mehreren Substituenten substituiert ist, die unabhängig voneinander aus einem Halogenatom und den Resten -OH, Amino, Cyano und Aryl ausgewählt sind;
R₉ einen Rest Alkyl, Halogenalkyl, Cycloalkyl oder Cycloalkylalkyl oder einen der Reste carbocyclisches oder heterocyclisches Aralkyl oder Aryl darstellt, deren aromatischer Ring gegebenenfalls 1 bis 3 mal mit Substituenten substituiert ist, die unabhängig voneinander aus einem Halogenatom und einem Alkyl- oder Alkoxyrest ausgewählt sind;
R₁₀ ein Wasserstoffatom oder einen Alkyl- oder Arylrest darstellt, der gegebenenfalls 1 bis 3 mal mit Substituenten substituiert ist, die unabhängig voneinander aus einem Halogenatom und einem Alkyl- oder Alkoxyrest ausgewählt sind,
R₁₁ ein Wasserstoffatom, einen Alkylrest, einen Cycloalkylrest, einen Cycloalkylalkylrest, einen Rest -C (O) R₁₃, einen Rest -C (O) OR₁₃, einen Rest -SO₂R₁₃, einen Rest -C(O)NHR₁₃ oder einen der Reste Aryl oder Aralkyl darstellt, deren aromatischer Ring gegebenenfalls 1 bis 3 mal mit Substituenten substituiert ist, die unabhängig voneinander aus einem Halogenatom und einem Alkyl- oder Alkoxyrest ausgewählt sind;
R₁₂ ein Wasserstoffatom oder einen Alkylrest darstellt;
R₁₃ einen Alkylrest, einen Halogenalkylrest oder einen der Reste carbocyclisches oder heterocyclisches Aralkyl oder Aryl darstellt, deren aromatischer Ring gegebenenfalls 1 bis 3 mal mit Substituenten substituiert ist, die unabhängig voneinander aus einem Halogenatom und einem Alkyl- oder Alkoxyrest ausgewählt sind;
R₁₄ einen Alkylrest, den Phenylrest oder einen Aralkylrest darstellt; und schließlich
R₁₅ einen Alkylrest, den Phenylrest oder einen Aralkylrest darstellt;
wobei gilt:
- dass ein Alkyl- oder Alkoxyrest, wenn es nicht genauer angegeben ist, linear oder verzweigt ist und 1 bis 12 Kohlenstoffatome zählt;
- dass ein Alkenyl- oder Alkinylrest, wenn es nicht genauer angegeben ist, linear oder verzweigt ist und 2 bis 6 Kohlenstoffatome zählt;
- dass ein Cycloalkylrest, wenn es nicht genauer angegeben ist, 3 bis 7 Kohlenstoffatome zählt;
oder ein Salz einer Verbindung der allgemeinen Formel (I).

2. Verbindung der allgemeinen Formel (I) nach Anspruch 1, **dadurch gekennzeichnet, dass** X eine Bindung oder einen linearen oder verzweigten Alkylenrest von 1 bis 5 Kohlenstoffatomen darstellt und Y einen Rest NR₄R₅ darstellt;
oder ein Salz dieser Verbindung.

3. Verbindung der allgemeinen Formel (I) nach Anspruch 1, **dadurch gekennzeichnet, dass** X eine Bindung oder einen linearen oder verzweigten Alkylenrest von 1 bis 5 Kohlenstoffatomen darstellt und Y einen Rest darstellt: 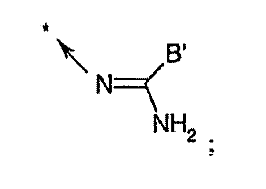 oder ein Salz dieser Verbindung.

4. Verbindung der allgemeinen Formel (I) nach Anspruch 1, **dadurch gekennzeichnet, dass** X eine Bindung oder einen linearen oder verzweigten Alkylenrest von 1 bis 5 Kohlenstoffatomen darstellt und Y einen Cycloalkylrest oder einen Arylrest darstellt, der gegebenenfalls 1 bis 3 mal mit Substituenten substituiert ist, die unabhängig voneinander aus einem Halogenatom und einem Alkyl- oder Alkoxyrest ausgewählt sind;
oder ein Salz dieser Verbindung.

5. Verbindung der allgemeinen Formel (I) nach Anspruch 1, **dadurch gekennzeichnet, dass** X eine Bindung darstellt und Y ein Wasserstoffatom darstellt, während mindestens einer der Reste R₁ und R₂ einen Rest darstellt, der aus den Resten Alkyl, Cycloalkyl oder Cycloalkylalkyl ausgewählt ist;
oder ein Salz dieser Verbindung.

6. Verbindung der allgemeinen Formel (I) nach Anspruch 1, **dadurch gekennzeichnet, dass** sie aus den folgenden Verbindungen ausgewählt ist:
- Butyl-2-[4-(4-{[(1Z)-amino(thien-2-yl)methylen]-amino}phenyl)-1*H*-imidazol-2-yl]ethylcarbamat ;
- Butyl-2-[4-(3-{[(1E)-amino(thien-2-yl)methylen]-amino}phenyl)-1*H*-imidazol-2-yl]ethylcarbamat;
- Butyl-2-[4-(4'-{[(1Z)-amino(thien-2-yl)methylen]amino}-1,1'-biphenyl-4-yl)-1*H*-imidazol-2-yl]ethylcarbamat; ,
- N'-(4-{2-[(Cyclohexylamino)methyl]-1*H*-imidazol-4-yl}phenyl)thiophen-2-carboximidamid;
- *N*'-(4-{2-[2-(Cyclohexylamino)ethyl]-1*H*-imidazol-4-yl}phenyl)thiophen-2-carboximidamid;
- *N*'-(3-{2-[(Cyclohexylamino)methyl]-1*H*-imidazol-4-yl}phenyl)thiophen-2-carboximidamid;
- *N*'-[4-(2-{[Cyclohexyl(methyl)amino]methyl}-1*H-*imidazol-4-yl)phenyl]thiophen-2-carboximidamid;
- *N*'-(4-{2-[(Dibenzylamino)methyl]-1*H*-imidazol-4-yl}phenyl)thiophen-2-carboximidamid;
- *N*'-(4-{2-[(Benzylamino)methyl]-1*H*-imidazol-4-yl}phenyl)thiophen-2-carboximidamid;
- *N*'-{3-[2-[(Aminomethyl)-1*H*-imidazol-4-yl}phenyl]thiophen-2-carboximidamid;
- *N*'-{3-[2-({[(1*E*)-Amino(thien-2-yl)methylen]-amino}methyl)-1*H*-imidazol-4-yl]phenyl}thiophen-2-carboximidamid;
- *N*'-{4-[2-({[(1*E*)-Amino(thien-2-yl)methylen]-amino}methyl)-1*H*-imidazol-4-yl]phenyl}thiophen-2-carboximidamid;
- *N*-{3-[2-(2-Cyclohexylethyl)-1*H*-imidazol-4-yl]phenyl}thiophen-2-carboximidamid;
- *N*'-{3-[2-(1-Pentylhexyl)-1*H*-imidazol-4-yl]phenyl}thiophen-2-carboximidamid;
- *N*'-{4-[2-(2-Cyclohexylethyl)-1*H*-imidazol-4-yl]phenyl}thiophen-2-carboximidamid;
- *N*'-{3-[2-(Cyclohexylmethyl)-1*H*-imidazol-4-yl]phenyl}thiophen-2-carboximidamid;
- *N*'-{3-[2-(3-Cyclohexylpropyl)-1*H*-imidazol-4-yl]phenyl}thiophen-2-carboximidamid;
- *N*'-[3-(2-Hexyl-1*H*-imidazol-4-yl]phenyl}thiophen-2-carboximidamid;
- *N*-{4-[2-(2-Cyclohexylethyl)-1*H*-imidazol-4-yl]-phenyl}-*N*"-nitroguanidin;
- *N*'-(4-{2-[(Cycloheptylamino)methyl]-1*H*-imidazol-4-yl}phenyl)thiophen-2-carboximidamid;
- *N*'-(4-{2-[(Methylamino)methyl]-1*H*-imidazol-4-yl}phenyl)thiophen-2-carboximidamid;
- *N*'-(4-{2-[(Cyclobutylamino)methyl]-1*H*-imidazol-4-yl}phenyl)thiophen-2-carboximidamid;
- *N*'-[4-(2-{[(2,2-Diphenylethyl)amino]methyl}-1*H-*imidazol-4-yl)phenyl]thiophen-2-carboximidamid;
- *N*'-{3-[2-(2-{[(1E)-Amino(thien-2-yl)methylen]amino}ethyl)-1H-imidazol-4-yl]phenyl}thiophen-2-carboximidamid;
- *N*'-(3-{2-[(Phenylthio)methyl]-1*H*-imidazol-4-yl}phenyl)thiophen-2-carboximidamid;
- *N*'-(4-{2-[(Phenylthio)methyl]-1*H*-imidazol-4-yl}phenyl)thiophen-2-carboximidamid;
- *N*'-{3-[2-(4-Isobutylbenzyl)-1*H*-imidazol-4-yl]phenyl}thiophen-2-carboximidamid;
oder ein Salz dieser Verbindung der allgemeinen Formel (I) .

7. Eine Verbindung der allgemeinen Formel (I), wie sie in Anspruch 1 definiert ist, oder ein pharmazeutisch annehmbares Salz dieser Verbindung als Medikament.

8. Pharmazeutische Zusammensetzung, die als Wirkstoff eine Verbindung der allgemeinen Formel (I), wie sie in Anspruch 1 definiert ist, oder ein pharmazeutisch annehmbares Salz dieser Verbindung enthält.

9. Verwendung einer Verbindung der allgemeinen Formel (I), wie sie in Anspruch 1 definiert ist, oder eines pharmazeutisch annehmbaren Salzes dieser Verbindung für die Herstellung eines Medikaments, das zur Behandlung oder Prävention einer Störung/Krankheit bestimmt ist, die aus den folgenden Störungen/Krankheiten ausgewählt ist: Schmerzen, Multiple Sklerose, Störungen des zentralen oder peripheren Nervensystems, kardiovaskuläre Störungen, Störungen der Skelettmuskulatur und der neuromuskulären Verbindungen, Entzündungskrankheiten, Hörverlust traumatischen, akustischen oder toxischen Ursprungs und Tinitus, mit Autoimmun- und Viruserkrankungen verbundene Komplikationen und neurologische Krankheiten in Verbindung mit Intoxikationen, mit Behandlungen oder mit Störungen genetischen Ursprungs.

10. Verwendung nach Anspruch 9, **dadurch gekennzeichnet, dass** das hergestellte Medikament dazu bestimmt ist, Schmerzen zu behandeln oder vorzubeugen.
